# EUROPEAN PATENT SPECIFICATION

(11) **EP 2 037 925 B1**
(45) Date of publication and mention of the grant of the patent: **01.02.2012**
(21) Application number: 07796424.5
(22) Date of filing: 26.06.2007
(51) Int. Cl.: A61K 31/495, A61K 45/06, A61P 25/28

(54) **COMBINATIONS COMPRISING 5HT6 MODULATORS AND CHOLINESTERASE INHIBITORS**
KOMBINATIONEN MIT 5HT6-MODULATOREN UND CHOLINESTERASE-HEMMERN
ASSOCIATIONS COMPRENANT DES MODULATEURS DE 5-HT6 ET DES INHIBITEURS DE CHOLINESTÉRASE

(30) Priority: 26.06.2006 US 816517 P
(43) Date of publication of application: 25.03.2009
(73) Proprietor: Galenea Corp., Cambridge MA 02139 (US)
(72) Inventor: GANNON, Kimberley, Watertown, MA 02472 (US); SHACHAM, Sharon, Newton, MA 02458 (US)
(74) Representative: Harris, Jennifer Lucy
(86) International application number: PCT/US2007/014747
(87) International publication number: WO 2008/002539

(56) References cited:
- WO-A-02/060871
- WO-A-2005/063761
- WO-A-2006/081332
- WO-A-2007/087151
- KING M V ET AL: "Combination of sub-threshold doses of Aricept and PRX-07034, a novel 5-HT6 receptor antagonist, enhances Novel Object Discrimination (NOD) memory" JOURNAL OF PHARMACOLOGICAL SCIENCES, JAPANESE PHARMACOLOGICAL SOCIETY, TOKYO, JP, vol. 101, no. Suppl 1, 2006, page 150, XP009086282 ISSN: 1347-8613
- LIEBEN C K J ET AL: "Cognitive effects of the acetylcholinesterase inhibitor metrifonate and the 5-HT6 antagonist RO4368554 in several rat models of memory deficiency in theobject recognition test" BEHAVIOURAL PHARMACOLOGY, RAPID SCIENCE, PUBLISHERS, GB, vol. 14, no. suppl 1, September 2003 (2003-09), page s31, XP009086240 ISSN: 0955-8810

## Description

### FIELD

The invention generally relates to the field of serotonin (5-hydroxytryptamine, or 5-HT) receptor modulators, e.g., 5-HT₆ antagonists, agonists, inverse agonists, or partial agonists, and more particularly to substituted arylamine compounds, in particular the use of these compounds with (acetyl)cholinesterase inhibitors and in pharmaceutical compositions, *e.g*., in the treatment, modulation and/or prevention of physiological or psychological conditions.

### BACKGROUND

The serotonergic receptors have been shown to influence a variety of physiologic functions which manifest themselves in a variety of disorders such as Alzheimer's disease, cognition disorders, irritable bowel syndrome, nausea, emesis, vomiting, prokinesia, gastroesophageal reflux disease, nonulcer dyspepsia, depression, anxiety, urinary incontinence, migraine, arrhythmia, atrial fibrillation, ischemic stroke, gastritis, gastric emptying disorders, feeding disorders, gastrointestinal disorders, constipation, erectile dysfunction, and respiratory depression.

Multiple serotonin (5-hydroxytryptamine or 5-HT) serotonin receptor subtypes have been identified and cloned. One of these, the 5-HT₆ receptor, has been cloned by several groups (*see, e.g.,* Ruat, M. et al. (1993) Biochem. Biophys. Res. Commun. 193: 268-276; Sebben, M. et al. (1994) NeuroReport 5: 2553-2557). The 5-HT₆ receptor (5-HT6R) is positively coupled to adenylyl cyclase and regulates several neurotransmitter systems including glutamate, aspartate and acetylcholine. 5-HT6Rs have been mainly localized in olfactory tubercles, striatum, nucleus accumbens, and hippocampus with lower levels also found in amygdala and hypothalamus. Compounds with enhanced affinity and selectivity for the 5-HT₆ receptor have been identified in a number of studies, *e.g.,* by Isaac, M. et al. (2000) Bioorganic & Medicinal Chemistry Letters 10: 1719-1721); and in patent publications such as WO 00/34242, WO99/37623, WO 99/42465, and WO 99/02502. 5-HT₆ mRNA appears to be almost exclusively present in the brain with little evidence for its presence in peripheral tissues. Therefore, 5-HT₆ antagonism has been proposed as a promising approach for treating cognitive impairment associated with neuropsychiatric disorders (e.g., Alzheimer's disease, schizophrenia) without having potential peripheral side effects.

Cognitive impairment is a debilitating feature of Alzheimer's disease and other neurologic and psychiatric disorders. Accumulating evidence supports the role of 5-HT6 receptors in cognition in a variety of animal models and cognitive assays, for example, 5-HT6 receptor blockade improved retention of spatial memory in the Morris water maze (Foley et al 2004, Neuropsychopharmacology 29:93-100; Rogers and Hagan 2001, Psychopharmacology 158:114-119; Stean et al 2002, Pharmacol Biochem Behav 71:645-54; Woolley et al 2001, Neuropharmacology 41:210-219), non-spatial recognition memory in novel object discrimination (NOD) (King et al 2004 Neuropharmacology 47:195-204; Schreiber et al 2007 Eur Neuropsychopharmacol 17:277-288) and reversal of scopolamine-induced deficits in various cognitive tasks (Lieben et al 2005, Neuropsychopharmacology 30:2169-2179; Woolley et al 2003, Psychopharmacology 170:358-367).

### SUMMARY

The present invention relates to the use of compositions or combinations which include 5-HT₆ modulators and cholinesterase or acetylcholinesterase inhibitors, for treating, preventing or curing Alzheimer's disease, memory conditions, cognition disorders, and depression. Such compositions include a 5-HT₆ modulator which may have the formula 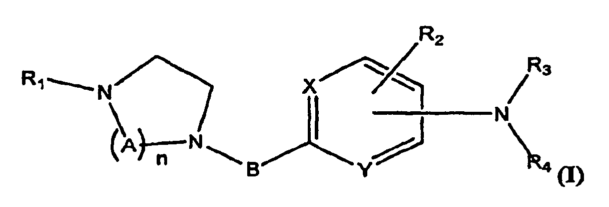 or the pharmaceutically acceptable salts and/or esters thereof, wherein n may be 0, 1, 2, 3, or 4; A, when present (*i.e*., n > 0) may be a lower alkyl, e.g., -CH₂-H₂ to form a piperazine, or-CH₂-CH₂-CH₂- to form an azapine ring; R₁ may be hydrogen or substituted or unsubstituted alkyl (*e.g*., lower alkyl) or aryl; R₂ may be hydrogen; halo; nitro; cyano, lower alkoxy; carboxylate salt acid or alkyl (*e.g*., lower alkyl) ester thereof, *e.g*., COR₅ where R₅ may be unsubstituted or mono-, di- or trisubstituted phenyl, biphenyl, heterocyclic, or a fused aromatic or heterocyclic ring, *e.g*., naphthyl or tetrahydronaphthyl; a sulfone (*e.g*., SO₂R₆ where R₆ may be, *e.g*., substituted or unsubstituted alkyl, haloalkyl, aryl, or heteroaryl); haloalkyl or haloalkoxy, *e.g*., mono-, di, or trifluoromethyl or methoxy; alkylamide; acetaldehyde; carboxamide; carbonyl; alkoxyaminocarbonyl; or substituted arylalkylamino; and R₃ and R₄ may independently be hydrogen, substituted or unsubstituted alkyl (*e.g*., lower alkyl), aryl (substituted or unsubstituted), alkylaryl, heteroaryl or alkylheteroaryl, or, taken together, R₃ and R₄ may form a substituted or unsubstituted aryl, alkylaryl, heteroaryl or alkylheteroaryl group, *e.g*., unsubstituted or mono-, di- or trisubstituted phenyl, biphenyl, or a fused aromatic or heterocyclic ring, *e.g*., naphthyl, tetrahydronaphthyl or benzothiophene; B may be absent or present, and when present may be a lower alkyl, *e.g*., methylene or a carbonyl group; and X and Y may each independently be C or N; and pharmaceutically acceptable salts and/or esters thereof; the first compound, in some embodiments, may be present in the composition in an amount which if administered alone does not substantially enhance memory. The composition also includes a second compound which is a cholinesterase or acetylcholinesterase inhibitor, which is present in an amount which, if administered to a patient alone, does not substantially enhance memory. The second compound is selected from the group consisting of: metrifonate, neostigmine, phydostigmine, pyridostigmine, galantamine/galanthamine, donepezil (Aricept®), tacrine, ambenonium, demarcarium, edrophonium, rivastigmine (Exelon®), phenserine, mentane, or eptastigmine; or pharmaceutically acceptable salts and/or esters thereof.

Compounds of the Formula I also include those where R₁ may be, *e.g*., H, CH₃, n-propyl, c-propyl, i-butyl, t-butyl, cyclohexyl, cyclohexylmethyl, phenyl or benzyl; A may be CH₂, (-CH₂-CH₂-), or (CH₂-CH₂-CH₂-); n may be 0, 1, 2, 3, or 4; B is absent; R₂ may be hydrogen; nitro; lower alkoxy; a sulfone (*e.g*., SO₂R₆ where R₆ may be, *e.g*., substituted or unsubstituted alkyl, haloalkyl, aryl, or heteroaryl); haloalkyl or haloalkoxy, *e.g*., mono-, di, or trifluoromethyl or methoxy; alkylamide; R₃ and R₄ independently may be lower alkyl or aryl or alkylaryl; and X and Y are both C.

In another embodiment, disclosed compositions include a first compound which is a 5-HT₆ modulator represented by formula II 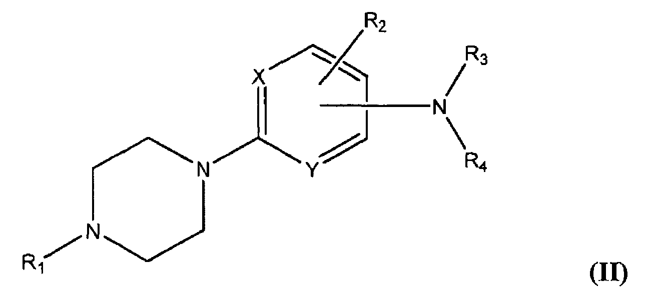 or pharmaceutically acceptable salts, and/or esters thereof, wherein R₁ may be hydrogen or substituted or unsubstituted alkyl (*e.g*., lower alkyl); R₂ may be hydrogen; halo; nitro; cyano, lower alkoxy; carboxylate salt acid or alkyl (*e.g*., lower alkyl) ester thereof; a sulfone (*e.g*., SO₂R₆ where R₆ may be, *e.g*., substituted or unsubstituted alkyl, haloalkyl, aryl, or heteroaryl); haloalkyl or haloalkoxy, *e.g*., mono-, di, or trifluoromethyl or methoxy; alkylamide; acetaldehyde; carboxamide; carbonyl; alkoxyaminocarbonyl; or substituted arylalkylamino; and R₃ and R₄ may independently be hydrogen, substituted or unsubstituted alkyl (*e.g*., lower alkyl) aryl (substituted or unsubstituted), alkylaryl, heteroaryl or alkylheteroaryl, or, taken together, R₃ and R₄ may form a substituted or unsubstituted aryl, alkylaryl, heteroaryl or alkylheteroaryl group, and a second compound which is present in an amount which, if administered to a patient alone, does not substantially enhance memory. The second compound is selected from the group consisting of: metrifonate, neostigmine, phydostigmine, pyridostigmine, galantamine/galanthamine, donepezil (Aricept®), tacrine, ambenonium, demarcarium, edrophonium, rivastigmine (Exelon®), phenserine, mentane, or eptastigmine; or pharmaceutically acceptable salts and/or esters thereof.

Contemplated compositions may include N-benzyl-3-piperazinyl benzenamine compounds represented by formula III: 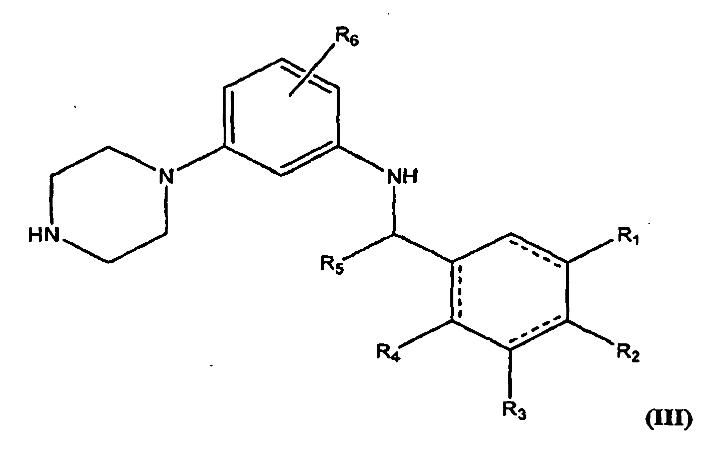 or pharmaceutically acceptable salts and/or esters thereof, wherein R₁, R₂, R₃ and R₄ may independently be hydrogen, halo or lower alkoxy, or two adjoining R₁, R₂, R₃ or R₄ lower alkoxy groups may, taken with the benzyl ring to which they are attached, combine to form a heterocyclic ring; R₅ may be hydrogen or lower alkoxy; and R₆ may be a sulfone (*e.g*., SO₂R where R may be, *e.g*., substituted or unsubstituted alkyl, haloalkyl, aryl, or heteroaryl); NO₂, or COCF₃. When two substituents are present on adjacent carbon atoms, the substituents, *e.g*., on an aromatic or carbocyclic ring, taken with the ring to which they are attached, may form a five to seven membered ring (for example, when the substituents are methoxy, a dioxane or dioxolane ring may be formed.

For example, contemplated compositions may include N-benzyl-3-piperazinyl compounds of the formula 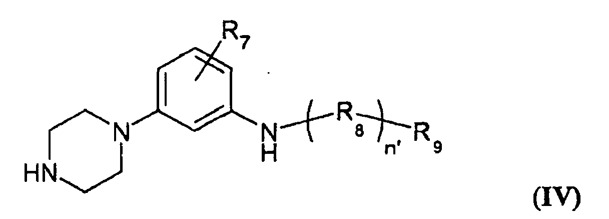 or pharmaceutically acceptable salts and/or esters thereof, wherein R₇ may be nitro; lower alkoxy, *e.g*., methyl; trihalo (*e.g*., trifluoro) methanone; sulfonyl; or alkyl (*e.g*., lower alkyl) sulfonyl. R₈ may be straight or branched C₁, C₂ or C₃ lower alkylene, and n' is 0, 1 or 2. R₉ is a single or conjugated cyclic ring, *e.g*., substituted or unsubstituted aryl, naphthyl, or chroman. Substituents on R₉ may include lower alkoxy, *e.g*., C₁, C₂ or C₃; halo; lower alkyl, *e.g.,* C₁, C₂ or C₃, R₉ may have more than one substituent, *e.g*., one, two, three or four. When two substituents are present on adjacent carbon atoms, the substituents, *e.g*., on an aromatic or carbocyclic ring, taken with the ring to which they are attached, may form a five to seven membered ring (for example, when the substituents are methoxy, a dioxane or dioxolane ring may be formed. Taken together, the two substituents may thus form, *e.g*., a substituted or unsubstituted aryl, alkylaryl, heteroaryl or alkylheteroaryl group such as unsubstituted or mono-, di- or trisubstituted phenyl, biphenyl, or a fused aromatic or heterocyclic rings, e.g., naphthyl or tetrahydronaphthyl or benzothiophene.

In an embodiment, disclosed compositions comprise between about 10 mg and about 100 mg of the first compound, or about 100 mg and about 1000 mg, or even about 1000 mg to about 3000 mg of the first compound. In another embodiment, disclosed compositions comprise between about 0.01 mg and about 4.5 mg of the second compound.

The invention also provides compositions of the invention for use in treating Alzheimer's disease or a cognition disorder. A patient in need thereof (*e.g*., one having been diagnosed as in need of treatment) may be administered a therapy including a first compound which is a 5-HT₆ modulator, e.g. a compound of Formula I, II, III, or IV and a second compound which is a cholinesterase or acetylcholinesterase inhibitor and which is present in an amount which, if administered to a patient alone, does not substantially enhance memory. The second compound is selected from the group consisting of: metrifonate, neostigmine, phydostigmine, pyridostigmine, galantamine/galanthamine, donepezil (Aricept®), tacrine, ambenonium, demarcarium, edrophonium, rivastigmine (Exelon®), phenserine, mentane, or eptastigmine; or pharmaceutically acceptable salts and/or esters thereof. The first compound may be present in an amount, if administered alone, does not substantially or optimally enhance memory. The first compound and the cholinesterase or acetylcholinesterase inhibitor may be administered substantially simultaneously, or sequentially and may be administered in separate dosage forms or as single dosage form.

In a particular embodiment, a composition is provided that includes donepezil hydrochloride and [1-(5-Chloro-2,3-dimethoxy-phenyl)-ethyl]-(2-methanesulfonyl-5-piperazin- 1-yl-phenyl)-amine, or a pharmaceutically acceptable salt and/or ester thereof.

### DETAILED DESCRIPTION

The features and other details of the invention will now be more particularly described. It will be understood that particular embodiments described herein are shown by way of illustration of the invention. The principal features of this invention can be employed in various embodiments. All parts and percentages are by weight unless otherwise specified.

### Definitions

For convenience, certain terms used in the specification, examples, and appended claims are collected here.

"5-HT₆ receptor modulator" includes compounds having effect at the 5-HT₆ receptor site, including subtypes of the receptor type. 5-HT₆ modulators may be agonists, partial agonists, inverse agonists, or antagonists.

"Treating", includes any effect, *e.g*., lessening, reducing, modulating, or eliminating, that results in the improvement of the condition, disease, disorder, etc.

"Alkyl" includes saturated aliphatic groups, including straight-chain alkyl groups (*e.g*., methyl, ethyl, propyl, butyl, pentyl, hexyl, heptyl, octyl, nonyl, decyl), branched-chain alkyl groups (*e.g*., isopropyl, tert-butyl, isobutyl), cycloalkyl (*e.g*., alicyclic) groups (*e.g*., cyclopropyl, cyclopentyl, cyclohexyl, cycloheptyl, cyclooctyl), alkyl substituted cycloalkyl groups, and cycloalkyl substituted alkyl groups. "Alkyl" further includes alkyl groups which have oxygen, nitrogen, sulfur or phosphorous atoms replacing one or more hydrocarbon backbone carbon atoms. In certain embodiments, a straight chain or branched chain alkyl has six or fewer carbon atoms in its backbone (*e.g*., C₁-C₆ for straight chain, C₃-C₆ for branched chain), and more preferably four or fewer. Likewise, preferred cycloalkyls have from three to eight carbon atoms in their ring structure, and more preferably have five or six carbons in the ring structure. "C₁-C₆" includes alkyl groups containing one to six carbon atoms.

The term "alkyl" also includes both "unsubstituted alkyls" and "substituted alkyls", the latter of which refers to alkyl moieties having substituents replacing a hydrogen on one or more carbons of the hydrocarbon backbone. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Cycloalkyls can be further substituted, *e.g*., with the substituents described above. An "alkylaryl" or an "aralkyl" moiety is an alkyl substituted with an aryl (*e.g*., phenylmethyl (benzyl)). "Alkyl" also includes the side chains of natural and unnatural amino acids.

"Aryl" includes groups with aromaticity, including 5- and 6-membered "unconjugated", or single-ring, aromatic groups that may include from zero to four heteroatoms, as well as "conjugated", or multicyclic, systems with at least one aromatic ring. Examples of aryl groups include benzene, phenyl, pyrrole, furan, thiophene, thiazole, isothiazole, imidazole, triazole, tetrazole, pyrazole, oxazole, isooxazole, pyridine, pyrazine, pyridazine, and pyrimidine, and the like. Furthermore, the term "aryl" includes multicyclic aryl groups, *e.g*., tricyclic, bicyclic, *e.g*., naphthalene, benzoxazole, benzodioxazole, benzothiazole, benzoimidazole, benzothiophene, methylenedioxyphenyl, quinoline, isoquinoline, napthridine, indole, benzofuran, purine, benzofuran, deazapurine, or indolizine. Those aryl groups having heteroatoms in the ring structure may also be referred to as "aryl heterocycles", "heterocycles," "heteroaryls" or "heteroaromatics". The aromatic ring can be substituted at one or more ring positions with such substituents as described above, as for example, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, aralkylaminocarbonyl, alkenylaminocarbonyl, alkylcarbonyl, arylcarbonyl, aralkylcarbonyl, alkenylcarbonyl, alkoxycarbonyl, aminocarbonyl, alkylthiocarbonyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Aryl groups can also be fused or bridged with alicyclic or heterocyclic rings which are not aromatic so as to form a multicyclic system (*e.g*., tetralin, methylenedioxyphenyl).

"Alkenyl" includes unsaturated aliphatic groups analogous in length and possible substitution to the alkyls described above, but that contain at least one double bond. For example, the term "alkenyl" includes straight-chain alkenyl groups (*e.g*., ethenyl, propenyl, butenyl, pentenyl, hexenyl, heptenyl, octenyl, nonenyl, decenyl), branched-chain alkenyl groups, cycloalkenyl (*e.g*., alicyclic) groups (*e.g*., cyclopropenyl, cyclopentenyl, cyclohexenyl, cycloheptenyl, cyclooctenyl), alkyl or alkenyl substituted cycloalkenyl groups, and cycloalkyl or cycloalkenyl substituted alkenyl groups. The term "alkenyl" further includes alkenyl groups which include oxygen, nitrogen, sulfur or phosphorous atoms replacing one or more hydrocarbon backbone carbons. In certain embodiments, a straight chain or branched chain alkenyl group has six or fewer carbon atoms in its backbone (*e.g*., C₂-C₆ for straight chain, C₃-C₆ for branched chain.) Likewise, cycloalkenyl groups may have from three to eight carbon atoms in their ring structure, and more preferably have five or six carbons in the ring structure. The term "C₂-C₆" includes alkenyl groups containing two to six carbon atoms.

The term "alkenyl" also includes both "unsubstituted alkenyls" and "substituted alkenyls", the latter of which refers to alkenyl moieties having substituents replacing a hydrogen on one or more hydrocarbon backbone carbon atoms. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

"Alkynyl" includes unsaturated aliphatic groups analogous in length and possible substitution to the alkyls described above, but which contain at least one triple bond. For example, "alkynyl," includes straight-chain alkynyl groups (*e.g*., ethynyl, propynyl, butynyl, pentynyl, hexynyl, heptynyl, octynyl, nonynyl, decynyl), branched-chain alkynyl groups, and cycloalkyl or cycloalkenyl substituted alkynyl groups. The term "alkynyl" further includes alkynyl groups having oxygen, nitrogen, sulfur or phosphorous atoms replacing one or more hydrocarbon backbone carbons. In certain embodiments, a straight chain or branched chain alkynyl group has six or fewer carbon atoms in its backbone (*e.g*., C₂-C₆ for straight chain, C₃-C₆ for branched chain). The term "C₂-C₆" includes alkynyl groups containing two to six carbon atoms.

The term "alkynyl" also includes both "unsubstituted alkynyls" and "substituted alkynyls", the latter of which refers to alkynyl moieties having substituents replacing a hydrogen on one or more hydrocarbon backbone carbon atoms. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

Unless the number of carbons is otherwise specified, "lower alkyl" includes an alkyl group, as defined above, but having from one to ten, more preferably from one to six, carbon atoms in its backbone structure. "Lower alkenyl" and "lower alkynyl" have chain lengths of, for example, 2-5 carbon atoms.

"Acyl" includes compounds and moieties which contain the acyl radical (CH₃CO-) or a carbonyl group. "Substituted acyl" includes acyl groups where one or more of the hydrogen atoms are replaced by for example, alkyl groups, alkynyl groups, halogens, hydroxyl, alkylcarbonyloxy, arylcarbonyloxy, alkoxycarbonyloxy, aryloxydarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

"Acylamino" includes moieties wherein an acyl moiety is bonded to an amino group. For example, the term includes alkylcarbonylamino, arylcarbonylamino, carbamoyl and ureido groups.

"Alkoxyalkyl", "alkylaminoalkyl" and "thioalkoxyalkyl" include alkyl groups, as described above, which further include oxygen, nitrogen or sulfur atoms replacing one or more hydrocarbon backbone carbon atoms, *e.g*., oxygen, nitrogen or sulfur atoms.

The term "alkoxy" includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, and tri chloromethoxy.

The terms "heterocyclyl" or "heterocyclic group" include closed ring structures, *e.g*., 3- to 10-, or 4- to 7-membered rings, which include one or more heteroatoms. Heterocyclyl groups can be saturated or unsaturated and include pyrrolidine, oxolane, thiolane, piperidine, piperizine, morpholine, lactones, lactams such as azetidinones and pyrrolidinones, sultams, sultones, and the like. The heterocyclic ring can be substituted at one or more positions with such substituents, as described above, as for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, sulfonato, sulfamoyl, sulfonamido, nitro, trifluoromethyl, cyano, azido, heterocyclyl, or an aromatic or heteroaromatic moiety.

The term "thiocarbonyl" or "thiocarboxy" includes compounds and moieties which contain a carbon connected with a double bond to a sulfur atom.

The term "ether" includes compounds or moieties which contain an oxygen bonded to two different carbon atoms or heteroatoms. For example, the term includes "alkoxyalkyl" which refers to an alkyl, alkenyl, or alkynyl group covalently bonded to an oxygen atom which is covalently bonded to another alkyl group.

The term "ester" includes compounds and moieties which contain a carbon or a heteroatom bound to an oxygen atom which is bonded to the carbon of a carbonyl group. The term "ester" includes alkoxycarboxy groups such as methoxycarbonyl, ethoxycarbonyl, propoxycarbonyl, butoxycarbonyl, pentoxycarbonyl, etc. The alkyl, alkenyl, or alkynyl groups are as defined above.

The term "thioether" includes compounds and moieties which contain a sulfur atom bonded to two different carbon or heteroatoms. Examples of thioethers include, but are not limited to alkthioalkyls, alkthioalkenyls, and alkthioalkynyls. The term "alkthioalkyls" include compounds with an alkyl, alkenyl, or alkynyl group bonded to a sulfur atom which is bonded to an alkyl group. Similarly, the term "alkthioalkenyls" and alkthioalkynyls" refer to compounds or moieties wherein an alkyl, alkenyl, or alkynyl group is bonded to a sulfur atom which is covalently bonded to an alkynyl group.

The term "hydroxy" or "hydroxyl" includes groups with an -OH or -O⁻.

The term "halogen" includes fluorine, bromine, chlorine, iodine, etc. The term "perhalogenated" generally refers to a moiety wherein all hydrogens are replaced by halogen atoms.

"Heteroatom" includes atoms of any element other than carbon or hydrogen. Examples of heteroatoms include nitrogen, oxygen, sulfur and phosphorus.

It will be noted that the structure of some of the compounds of the invention includes asymmetric carbon atoms. It is to be understood accordingly that the isomers arising from such asymmetry (*e.g*., all enantiomers and diastereomers) are included within the scope of the invention, unless indicated otherwise. Such isomers can be obtained in substantially pure form by classical separation techniques and by stereochemically controlled synthesis. Furthermore, the structures and other compounds and moieties discussed in this application also include all tautomers thereof. Alkenes can include either the E- or Z-geometry, where appropriate.

"Combination therapy" (or "co-therapy") includes the administration of a 5-HT modulator of the invention and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected). "Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. The sequence in which the therapeutic agents are administered is not narrowly critical. "Combination therapy" also can embrace the administration of the therapeutic agents as described above in further combination with other biologically active ingredients and non-drug therapies (*e.g*., surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

The term "heterocyclic group" is intended to include closed ring structures in which one or more of the atoms in the ring is an element other than carbon, for example, nitrogen, or oxygen or sulfur. Heterocyclic groups can be saturated or unsaturated and heterocyclic groups such as pyrrole and furan can have aromatic character. They include fused ring structures such as quinoline and isoquinoline. Other examples of heterocyclic groups include pyridine and purine. Heterocyclic groups can also be substituted at one or more constituent atoms with, for example, a halogen, a lower alkyl, a lower alkenyl, a lower alkoxy, a lower alkylthio, a lower alkylamino, a lower alkylcarboxyl, a nitro, a hydroxyl, -CF₃, -CN, or the like.

The terms "individual," "patient," or "subject" are used interchangeably herein and include any mammal, including animals, for example, primates, for example, humans, and other animals, for example, dogs, cats, swine, cattle, sheep, and horses. The compounds of the invention can be administered to a mammal, such as a human, but can also be other mammals, for example, an animal in need of veterinary treatment, for example, domestic animals (for example, dogs, cats, and the like), farm animals (for example, cows, sheep, pigs, horses, and the like) and laboratory animals (for example, rats, mice, guinea pigs, and the like).

The phrase "minimizing adverse effects," "reducing adverse events," or "reduced adverse events," as used herein refer to an amelioration or elimination of one or more undesired side effects associated with the use of acetylcholinesterase or cholinesterase inhibitors. Such side effects include, without limitation, diarrhea, anorexia, vomiting, nausea, and ecchymosis.

In certain embodiments, side effects of administering certain compounds may be partially eliminated. As used herein, the phrase "partially eliminated" refers to a reduction in the severity, extent, or duration of the particular side effect by at least 30%, 40%, 50%, 60%, 70%, 80%, 90%, 95% and 99% relative to that found by administering, for example, 10 mg/day of donepezil monotherapy. Those skilled in the art are credited with the ability to detect and grade the severity, extent, or duration of side effects as well as the degree of amelioration of a side effect. In some embodiments, two or more side effects are ameliorated.

The term "synergistic" refers to two or more agents, e.g. a disclosed compound of Formula I, II or II and an acetylcholinesterase or cholinesterase inhibitor, when taken together, produce a total joint effect that is greater than the sum of the effects of each drug when taken alone.

### Methods

The disclosed compositions and/or combinations of compounds, in certain embodiments, can be used for treating a wide variety of clinical conditions which may be characterized by acetylcholine hypofunction. Such conditions include Alzheimer's disease as well as a variety of disorders associated with cognitive impairment such as dementia, amnestic and other cognitive or neurodegenerative disorders, such as senile dementia, dementia of the Alzheimer's type, vascular dementia, and other dementias, for example, due to HIV disease, head trauma, Parkinson's disease, Huntington's disease, Pick's disease, Creutzfeldt-Jakob disease, or due to multiple etiologies; schizophrenia and other psychotic disorders, for example, schizophrenic disorders, schizoaffective disorders, delusional disorders, brief psychotic disorders, shared psychotic disorders and psychotic disorders with delusions or hallucinations; addiction disorders; obsessive compulsive disorders, panic disorders, sleep disorders, alcoholism, pain, memory deficits, unipolar depression, dysthymia, bipolar depression, treatment-resistant depression, depression in the medically ill, panic disorder, obsessive-compulsive disorder, social phobia, premenstrual dysphoric disorder, mood disorders, such as depression or more particularly depressive disorders, for example, single episodic or recurrent major depressive disorders and dysthymic disorders, or bipolar disorders, for example, bipolar I disorder, bipolar II disorder and cyclothymic disorder; anxiety disorders, such as panic disorder with or without agoraphobia, agoraphobia without history of panic disorder, specific phobias, *e.g*., specific animal phobias, social phobias, stress disorders including post-traumatic stress disorder and acute stress disorder, and generalized anxiety disorders; delirium, Parkinson's disease and other extra-pyramidal movement disorders such as medication-induced movement disorders, for example, neuroleptic-induced parkinsonism, neuroleptic malignant syndrome, neuroleptic-induced acute dystonia, neuroleptic-induced acute akathisia, neuroleptic-induced tardive dyskinesia and medication-induced postural tremor; substance-related disorders arising from the use of alcohol, amphetamines (or amphetamine-like substances) caffeine, cannabis, cocaine, hallucinogens, inhalants and aerosol propellants, nicotine, opioids, phenylglycidine derivatives, sedatives, hypnotics, and anxiolytics, which substance-related disorders include dependence and abuse, intoxication, withdrawal, intoxication delirium, withdrawal delirium, persisting dementia, psychotic disorders, mood disorders, anxiety disorders, sexual dysfunction and sleep disorders; epilepsy; Down's syndrome; demyelinating diseases such as MS and ALS and other neuropathological disorders such as peripheral neuropathy, for example, diabetic and chemotherapy-induced neuropathy, and postherpetic neuralgia, trigeminal neuralgia, segmental or intercostal neuralgia and other neuralgias; and cerebral vascular disorders due to acute or chronic cerebrovascular damage such as cerebral infarction, subarachnoid hemorrhage or cerebral edema. In certain embodiments, methods disclosed herein include administrating to a patient in need thereof disclosed compositions or combinations, e.g. a 5-HT₆ modulator and a cholinesterase inhibitor. Such a combination of a 5-HT₆ modulator and a cholinesterase inhibitor, may, in some embodiments, provide additive and/or synergistic effects.

In some embodiments, a composition is provided for use in treating Alzheimer's disease, cognition disorders, and/or for enhancing memory in a patient in need thereof. A patient in need thereof is administered a first compound represented by formulae I, II, III or IV in combination with a cholinesterase or acetylcholinesterase inhibitor. The first compound and/or the cholinesterase or acetylcholinesterase inhibitor may be present in an amount which, if each amount is administered alone, would not substantially enhance memory in the patient.

For example, the cholinesterase or acetylcholinesterase inhibitor may be donepezil or donepezil hydrochloride and may be administered at a dosage between about 0.01 mg/day and 4.5 mg/day. Alternatively, the cholinesterase or acetylcholinesterase inhibitor may be galantamine or galantamine hydrobromide can be administered at a dosage between about 0.01 mg and 7.5 mg, e.g. twice daily. In another example the cholinesterase or acetylcholinesterase inhibitors rivastigmine or rivastigmine tartrate may be administered at a dosage between about 0.01 mg and 1.0 mg, e.g. twice daily.

When treating Alzheimer's disease, a cognition disorder, and/or enhancing memory in a patient with a composition of the invention, the relevant disorder may be treated, but with a reduced incidence of an adverse event as compared to administration of an effective amount of an acetylcholinesterase inhibitor or a cholinesterase inhibitor alone, e.g. as compared to a dosage of 10 mg/day or 5 mg/day of an acetylcholinesterase alone, e.g. donepezil hydrochloride during monotherapy.

### Compositions and Dosages

Compositions and combinations of compounds disclosed herein may be used for the treatment of the conditions related to the central nervous system, as well as for the particular conditions indicated above. For treating certain conditions it may be desirable to employ a 5-HT₆ modulator such as the disclosed compounds of e.g. Formula I, II or III, represented above, in conjunction with another pharmacologically active agent, e.g. an acetylcholinesterase inhibitor. The compounds of the invention may be presented together with another therapeutic agent as a combined preparation for simultaneous, separate or sequential use.

A disclosed composition may include at least one first compound or agent chosen from those compounds represented by Formula I, II or III, above, e.g., a 5-HT₆ modulator, and a second compound chosen from a cholinesterase or acetylcholinestase inhibitor. Such 5-HT₆ modulator and a cholinesterase or acetylcholinesterase inhibitor can be administered as (i) a single dosage form or composition, (ii) simultaneously as separate dosage forms or pharmaceutical compositions, (iii) sequentially, as separate dosage forms starting with the 5-HT₆ modulator and then administering the cholinesterase or acetylcholinesterase inhibitor, or starting with the cholinesterase or acetylcholinesterase inhibitor and then administering the 5-HT₆ modulator, (iv) successively, separated by for example 1-4 hours, 1-8 hours or 1-12 hours, a day, or 2 or more days, (v) individually followed by the combination. It will be appreciated that when using a combination or composition of the invention, the 5-HT₆ modulator and the other pharmacologically active agent may be in the same pharmaceutically acceptable carrier and therefore administered simultaneously. They may be in separate pharmaceutical carriers such as conventional oral dosage forms which can be taken simultaneously, and can e.g. be presented in the form of a twin pack.

Disclosed combinations may be administered to patients (animals and humans) in need of such treatment in dosages that will provide optimal pharmaceutical efficacy. It will be appreciated that the dose required for use in any particular application will vary from patient to patient, not only with the particular compound or composition selected, but also with the route of administration, the nature of the condition being treated, the age and condition of the patient, concurrent medication or special diets then being followed by the patient, and other factors which those skilled in the art will recognize, with the appropriate dosage ultimately being at the discretion of the attendant physician.

Contemplated cholinesterase or acetylcholinesterase inhibitors are metrifonate, neostigmine, phydostigmine, pyridostigmine, galantamine/galanthamine, donepezil (Aricept®), tacrine, ambenonium, demarcarium, edrophonium, rivastigmine (Exelon®), phenserine, mentane, or eptastigmine; or pharmaceutically acceptable salts and/or esters thereof.

Contemplated 5-HT₆ modulators of a disclosed combination or composition include [1 -(5-Chloro-2,3-dimethoxy-phenyl)-ethyl]-(2-methanesulfonyl-5-piperazin-1-yl-phenyl)-amine, (2-(methylsulfonyl)-N-(I-[rho]henylethyl)-5-(piperazin-1-yl)benzenamine, (1-(2-(1-(3,5-dimethoxyphenyl)ethylamino)-4-(piperazin-1-yl)phenyl)-2,2,2-trifluoroethanone, N-(1-(6-Chloro-2,3-dihydrobenzo[b][1,4]dioxin-8-yl)ethyl)-2-(methylsulfonyl)-5-(piperazin-1-yl)benzenamine, N-(3-chlorobenzyl)-2-nitro-5-(piperazin-1-yl)benzenamine, N-(3-chlorobenzyl)-4-nitro-3-(piperazin-1-yl)benzenamine, 1-(2-(I-(3,5-dimethoxyphenyl)ethylamino)-4-(piperazin-1-yl)phenyl)-2,2,2-trifluoroethanone, N-(1-(3-chlorophenyl)ethyl)-2-nitro-5-(piperazin-1-yl)benzenarnine, N-(I-(3,5-dimethoxyphenyl)ethyl)-2-nitro-5 -(piperazin-1-yl)benzenamine, 1-(2-(1-Phenylethylamino)-4-(piperazin-1-yl)phenyl)-2,2,2-trifluoroethanone, 1-(2-(1-(3,5-dimethoxyphenyl)ethylamino)-4-(piperazin-1-yl)phenyl)-2,2,2-trifluoroethanone, N-(1-(3,5-Dimethoxyphenyl)ethyl)-2-nitro-5-(piperazin-1-yl)benzenamine, 1-(2-(I-Phenylethylamino)-4-(piperazin-1-yl)phenyl)-2,2,2-trifluoroethanone, 1-(2-(1-(3,5-dimethoxyphenyl)ethylamino)-4-(piperazin-1-yl)phenyl)-2,2,2-trifluoroethanone, 1-(2-(1-(3-methoxyphenyl)ethylamino)-4-(piperazin-1-yl)phenyl)-2,2,2-trifluoroethanone, 1-(2-(1-(3,5-Dichlorophenyl)ethylamino)-4-(piperazin-1-yl)phenyl)-2,2,2-trifluoroethanone,N-(1-(5-Chloro-2-methoxyphenyl)ethyl)-2-(methylsulfonyl)-5-(piperazin-1-yl)benzenamine, N-(2-Methyl-1-phenylpropyl)-2-nitro-5-(piperazin-1-yl)benzenamine, 1-(2-(3,5-Dimethoxybenzylamino)-4-(piperazin-1-yl)phenyl)-2,2,2-trifluoroethanone, N-(1-(3-Bromophenyl)ethyl)-2-nitro-5-(piperazin-1-yl)benzenamine, 4-Methoxy-N-(1-phenyl)ethyl)-3-(piperazin-1-yl)benzenamine, N-(3-Bromobenzyl)-2-nitro-5-(piperazin-1-yl)benzenamine, or a pharmaceutically acceptable salt and/or ester thereof, N-(1-(3,5-Dimethylyphenyl)ethyl)-2-nitro-5-(piperazin-1-yl)benzenamine, 1-(2-(3-bromobenzylamino)-4-(piperazin-1-yl)phenyl)-2,2,2 -trifluoroethanone, (S)-1-(2-(1-Phenylethylamino)-4-(piperazin-1-yl)phenyl)-2,2,2-trifluoroethanone, (R)-1-(2-(1-Phenylethylamino)-4-(piperazin-1-yl)phenyl)-2,2,2-trifluoroethanone, N-(2-(Methylsulfonyl)-5-(piperazin-1-yl)phenyl)naphthalen-1-amine hydrochloride, or N-(5-Fluoro-2-(methylsulfonyl)phenyl)-1,2,3,4-tetrahydronaphthalen-1-amine, pharmaceutically acceptable salts and/or esters thereof.

An appropriate dosage level for the disclosed compounds or compositions may be generally be about 0.001 to 50 mg per kg patient body weight per day, which may be administered in single or multiple doses. In some embodiments, the dosage level will be about 0.01 to about 25 mg/kg per day; e.g., about 0.05 to about 10 mg/kg per day. For example, in the treatment or prevention of a disorder of the central nervous system, a suitable dosage level may be about 0.001 to 10 mg/kg per day, e.g. about 0.005 to 5 mg/kg per day, or even about 0.01 to 1 mg/kg per day. The compounds may be administered on a regimen of 1 to 4 times per day, preferably once or twice per day.

In some embodiments, the disclosed combinations and compositions include a 5-HT₆ modulator, or first compound. For example, the disclosed combinations and/or composition may include a dosage amount of between about 10 and about 100 mg of a 5-HT₆ modulator, e.g., 10mg, 50 mg, or 100 mg; between about 100 mg and about 1000 mg, e.g. 250 mg, 400 mg, or 700 mg; between about 50 mg and 250 mg; between about 250 mg and 800mg; or even between about 1000 mg to about 3000mg.

Disclosed combinations may include a non-efficacious amount of a cholinesterase inhibitor, e.g. non-efficacious for cognitive enhancement and/or disease modification. For example, a disclosed combination or composition may include a dose amount of a cholinesterase inhibiter, e.g. donepezil, that is less than about 5 mg, *e.g*., between 0.01 and 4.5mg, or 1mg to about 4 mg. An acetylcholinesterase or cholinesterase inhibitor may be present in an amount that is sufficient to provide to a patient less than about 0.5mg/kg, e.g., between about 0.05 and about 0.5 mg/kg.

For example, a composition or combination may include between about 10 mg to about 550 mg of a 5-HT₆ modulator, e.g. [1-(5-Chloro-2,3-dimethoxy-phenyl)-ethyl]-(2-methanesulfonyl-5-piperazin-1-yl-phenyl)-amine or pharmaceutically acceptable salt thereof and between about 0.01 and 4.5 mg of donepezil or a pharmaceutically acceptable salt thereof.

In a different embodiment, a combination or composition may include a 5-HT₆ modulator and an amount of cholinesterase inhibitor (e.g., donepezil) that is sufficient to provide a dosage of about 5 mg or greater, *e.g*., between about 5 and 30 mg, or a dose or amount of a cholinesterase inhibitor which is efficacious or optimal in cognitive enhancement and/or disease modification in monotherapy.

### Formulation and Administration

The compositions and combination therapies of the invention may be administered in combination with a variety of pharmaceutical excipients, including stabilizing agents, carriers and/or encapsulation formulations as described herein.

Aqueous compositions of the present invention comprise an effective amount of the peptides of the invention, dissolved or dispersed in a pharmaceutically acceptable carrier or aqueous medium.

"Pharmaceutically or pharmacologically acceptable" refer to molecular entities and compositions that do not produce an adverse, allergic or other untoward reaction when administered to an animal, or a human, as appropriate. "Pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

For human administration, preparations should meet sterility, pyrogenicity, general safety and purity standards as required by FDA Office of Biologies standards.

The pharmaceutical compositions of this invention may be used in the form of a pharmaceutical preparation, for example, in solid, semisolid or liquid form, which contains one or more of the compound of the invention, as an active ingredient, in admixture with an organic or inorganic carrier or excipient suitable for external, enteral or parenteral applications. The active ingredient may be compounded, for example, with the usual non- toxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an amount sufficient to produce the desired effect upon the process or condition of the disease.

For preparing solid compositions such as tablets, the principal active ingredient is mixed with a pharmaceutical carrier, *e.g*., conventional tableting ingredients such as corn starch, lactose, sucrose, sorbitol, talc, stearic acid, magnesium stearate, dicalcium phosphate or gums, and other pharmaceutical diluents, *e.g*., water, to form a solid preformulation composition containing a homogeneous mixture of a compound of the invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compositions of the invention may be incorporated for administration orally or by injection include aqueous solution, suitably flavored syrups, aqueous or oil suspensions, and emulsions with acceptable oils such as cottonseed oil, sesame oil, coconut oil or peanut oil, or with a solubilizing or emulsifying agent suitable for intravenous use, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatin.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulized by use of inert gases. Nebulized solutions may be breathed directly from the nebulizing device or the nebulizing device may be attached to a face mask, tent or intermittent positive pressure breathing machine. Solution, suspension or powder compositions may be administered, preferably orally or nasally, from devices which deliver the formulation in an appropriate manner.

For treating clinical conditions and diseases noted above, the compound of this invention may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques.

The preparation of an aqueous composition that contains a composition of the invention or an active component or ingredient will be known to those of skill in the art in light of the present disclosure. Typically, such compositions can be prepared as injectables, either as liquid solutions or suspensions; solid forms suitable for using to prepare solutions or suspensions upon the addition of a liquid prior to injection can also be prepared; and the preparations can also be emulsified.

Pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi.

Solutions of active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

Pharmaceutically acceptable salts include acid addition salts and which are formed with inorganic acids such as, for example, hydrochloric, hydrobromic, boric, phosphoric, sulfuric acids or phosphoric acids, or such organic acids as acetic, oxalic, tartaric, maleic, fumaric, citric, succinic, mesylic, mandelic, succinic, benzoic, ascorbic, methanesulphonic, a-keto glutaric, a-glycerophosphoric, glucose-1-phosphoric acids and the like. Salts formed with the free carboxyl groups can also be derived from inorganic bases such as, for example, sodium, potassium, ammonium, calcium, magnesium, or ferric hydroxides, and such organic bases as isopropylamine, trimethylamine, histidine, procaine and the like. Other examples of pharmaceutically acceptable salts include quaternary derivatives of the compounds of Formulae I, II, III or IV such as the compounds quaternized by compounds Rₓ-T wherein Rₓ is C₁₋₆ alkyl, phenyl-C₁₋₆ alkyl or C₅₋₇ cycloalkyl, and T is a radical corresponding to an anion of an acid. Suitable examples of Rₓ include methyl, ethyl and n- and iso-propyl; and benzyl and phenethyl. Suitable examples of T include halide, *e.g*., chloride, bromide or iodide. Yet other examples of pharmaceutically acceptable salts also include internal salts such as N-oxides.

Therapeutic or pharmacological compositions of the present invention will generally comprise an effective amount of the component(s) of the combination therapy, dissolved or dispersed in a pharmaceutically acceptable medium. Pharmaceutically acceptable media or carriers include any and all solvents, dispersion media, coatings, antibacterial and antifungal agents, isotonic and absorption delaying agents and the like. The use of such media and agents for pharmaceutical active substances is well known in the art. Supplementary active ingredients can also be incorporated into the therapeutic compositions of the present invention.

The preparation of pharmaceutical or pharmacological compositions will be known to those of skill in the art in light of the present disclosure. Typically, such compositions may be prepared as injectables, either as liquid solutions or suspensions; solid forms suitable for solution in, or suspension in, liquid prior to injection; as tablets or other solids for oral administration; as time release capsules; or in any other form currently used, including cremes, lotions, mouthwashes, inhalants and the like.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze drying techniques which yield a powder of the active ingredient plus any additional desired ingredient from a previously sterile-filtered solution thereof.

The preparation of more, or highly, concentrated solutions for direct injection is also contemplated, where the use of DMSO as solvent is envisioned to result in extremely rapid penetration, delivering high concentrations of the active agents to a small area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure.

In addition to the compounds formulated for parenteral administration, such as intravenous or intramuscular injection, other pharmaceutically acceptable forms include, e.g., tablets or other solids for oral administration; liposomal formulations; time-release capsules; and any other form currently used, including creams.

The use of sterile formulations, such as saline-based washes, by surgeons, physicians or health care workers to cleanse a particular area in the operating field may also be particularly useful. Therapeutic formulations in accordance with the present invention may also be reconstituted in the form of mouthwashes, or in conjunction with antifungal reagents. Inhalant forms are also envisioned. The therapeutic formulations of the invention may also be prepared in forms suitable for topical administration, such as in cremes and lotions.

Suitable preservatives for use in such a solution include benzalkonium chloride, benzethonium chloride, chlorobutanol, thimerosal and the like. Suitable buffers include boric acid, sodium and potassium bicarbonate, sodium and potassium borates, sodium and potassium carbonate, sodium acetate, sodium biphosphate and the like, in amounts sufficient to maintain the pH at between about pH 6 and pH 8, and preferably, between about pH 7 and pH 7.5. Suitable tonicity agents are dextran 40, dextran 70, dextrose, glycerin, potassium chloride, propylene glycol, sodium chloride, and the like, such that the sodium chloride equivalent of the ophthalmic solution is in the range 0.9 plus or minus 0.2%. Suitable antioxidants and stabilizers include sodium bisulfite, sodium metabisulfite, sodium thiosulfite, thiourea and the like. Suitable wetting and clarifying agents include polysorbate 80, polysorbate 20, poloxamer 282 and tyloxapol. Suitable viscosity-increasing agents include dextran 40, dextran 70, gelatin, glycerin, hydroxyethylcellulose, hydroxmethylpropylcellulose, lanolin, methylcellulose, petrolatum, polyethylene glycol, polyvinyl alcohol, polyvinylpyrrolidone, carboxymethylcellulose and the like.

Upon formulation, therapeutics will be administered in a manner compatible with the dosage formulation, and in such amount as is pharmacologically effective. The formulations are easily administered in a variety of dosage forms, such as the type of injectable solutions described above, but drug release capsules and the like can also be employed.

In this context, the quantity of active ingredient and volume of composition to be administered depends on the host animal to be treated. Precise amounts of active compound required for administration depend on the judgment of the practitioner and are peculiar to each individual.

A minimal volume of a composition required to disperse the active compounds is typically utilized. Suitable regimes for administration are also variable, but would be typified by initially administering the compound and monitoring the results and then giving further controlled doses at further intervals. For example, for parenteral administration, a suitably buffered, and if necessary, isotonic aqueous solution would be prepared and used for intravenous, intramuscular, subcutaneous or even intraperitoneal administration. One dosage could be dissolved in 1 ml of isotonic NaCl solution and either added to 1000 ml of hypodermolysis fluid or injected at the proposed site of infusion, (see for example, Remington's Pharmaceutical Sciences 15th Edition, pages 1035-1038 and 1570-1580).

In certain embodiments, active compounds may be administered orally. This is contemplated for agents which are generally resistant, or have been rendered resistant, to proteolysis by digestive enzymes. Such compounds are contemplated to include chemically designed or modified agents; dextrorotatory peptides; and peptide and liposomal formulations in time release capsules to avoid peptidase and lipase degradation.

The carrier can also be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Additional formulations suitable for other modes of administration include suppositories. For suppositories, traditional binders and carriers may include, for example, polyalkylene glycols or triglycerides; such suppositories may be formed from mixtures containing the active ingredient in the range of 0.5% to 10%, preferably 1%-2%.

Oral formulations include such normally employed excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations or powders.

In certain defined embodiments, oral pharmaceutical compositions will comprise an inert diluent or assimilable edible carrier, or they may be enclosed in hard or soft shell gelatin capsule, or they may be compressed into tablets, or they may be incorporated directly with the food of the diet. For oral therapeutic administration, the active compounds may be incorporated with excipients and used in the form of ingestible tablets, buccal tables, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 75% of the weight of the unit, or preferably between 25-60%. The amount of active compounds in such therapeutically useful compositions is such that a suitable dosage will be obtained.

The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup of elixir may contain the active compounds sucrose as a sweetening agent methyl and propylparabens as preservatives, a dye and flavoring, such as cherry or orange flavor.

5-HT₆ compounds used in the invention may be made as shown in pending U.S. Patent Application No. 11/340,079, filed on January 25, 2006.

The following example is intended to illustrate the compositions and uses of this invention. Many other embodiments will be apparent to one skilled in the art.

### EXAMPLE 1

This example demonstrates a combination therapy including [1-(5-Chloro-2,3-dimethoxy-phenyl)-ethyl]-(2-methanesulfonyl-5-piperazin-1-yl-phenyl)-amine hydrochloride 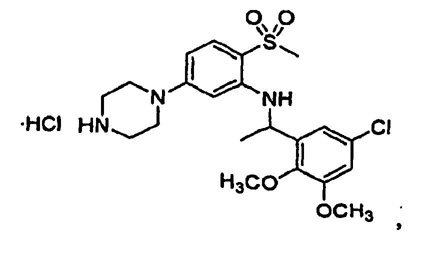 a 5-HT₆ receptor modulator compound A"), and donepezil (Aricept^{®}), a cholinesterase inhibitor, both in suboptimal doses if administered alone, in combination at these doses reverses memory deficits in the novel object discrimination (NOD) task, a test for determining memory enhancement.

The individual effects of Compound A (Kᵢ=4nM) and donepezil (Aricept^{®}) were characterized in this task, and determined the combined effect on memory of sub-maximal doses of each. NOD testing was performed as described by King *et al.* (2004 Neuropharmacol. 47 195), and used five separate groups (n=12) of adult male Lister hooded rats (145-235g). Within each group rats were tested on 3-4 occasions (at one-week intervals) to receive all possible treatment combinations and serve as their own controls.

A 4h inter-trial interval ensured natural forgetting in all groups following vehicle administration (water 1ml/kg i.p.), such that rats failed to discriminate the novel from the familiar object (p>0.05 in each case; Student's paired t-test). Compound A (40min pre-treatment) prevented this natural forgetting at doses of 3 and 10mg/kg i.p. (novel versus familiar p<0.001 & 0.01 respectively). These doses also increased the discrimination ratio from vehicle levels (p<0.05; repeated measures ANOVA). Aricept (20min pre-treatment) lacked effect at doses of 0.1 and 0.3mg/kg i.p., but prevented natural forgetting at a dose of 1 mg/kg i.p. (novel versus familiar p<0.001) without significantly increasing the discrimination ratio. Doses of Compound A that lacked significant effect when given alone (0.3 and 1 mg/kg i.p.) restored discrimination when combined with a sub-efficacious dose (0.1mg/kg i.p.) of Aricept (novel versus familiar p<0.01 & 0.05, respectively).

These data suggest a cognitive enhancing effect of not only compounds of the invention in the NOD task, but also, the effect of sub-threshold doses of compounds of the invention and (acetyl)cholinesterase inhibitors like Aricept may be of relevance for treating cognitive dysfunctions, such as that occurring in Alzheimer's disease.

### EQUIVALENTS

Those skilled in the art will recognize, or be able to ascertain using no more than routine experimentation, numerous equivalents to the specific procedures described herein. Various substitutions, alterations, and modifications may be made to the invention.

## Claims

1. A pharmaceutical composition comprising
• a first compound having the formula 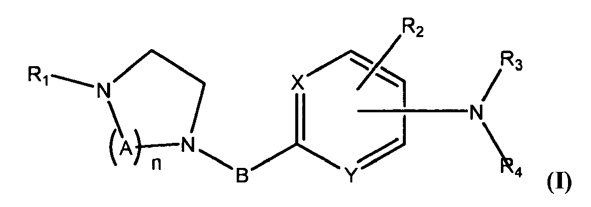 and pharmaceutically acceptable salts and/or esters thereof, wherein
- n is 0, 1, 2, 3, or 4;
- A, when present is a C₁₋₁₀ alkyl group;
- R₁ is hydrogen or substituted or unsubstituted alkyl or aryl;
- R₂ is hydrogen; halo; nitro; cyano, lower alkoxy; carboxylate salt acid or alkyl ester thereof; a sulfone; haloalkyl or haloalkoxy; acetaldehyde; carboxamide; carbonyl; alkoxyaminocarbonyl; or substituted arylalkylamino, wherein lower alkoxy is C₁₋₁₀ alkyl, C₂₋₅ alkenyl or C₂₋₅ alkynyl covalently linked to an oxygen atom;
- R₃ and R₄ independently are hydrogen, substituted or unsubstituted alkyl, aryl, alkylaryl, heteroaryl or alkylheteroaryl, or, taken together, R₃ and R₄ form one substituted or unsubstituted aryl, alkylaryl, heteroaryl or alkylheteroaryl group;
- B may be present or absent and, when present, is C₁₋₁₀ alkyl; and
- X and Y are independently C or N; and
a second compound selected from the group consisting of metrifonate, neostigmine, physostigmine, pyridostigmine, galantamine/galanthamine, donepezil, tacrine, ambenonium, demarcarium, edrophonium, rivastigmine, phenserine, mentane, and eptastigmine; or pharmaceutically acceptable salts and/or esters thereof, wherein the second compound is present in an amount which, if administered to a patient alone, does not enhance memory.

2. A pharmaceutical composition comprising
a first compound having the formula 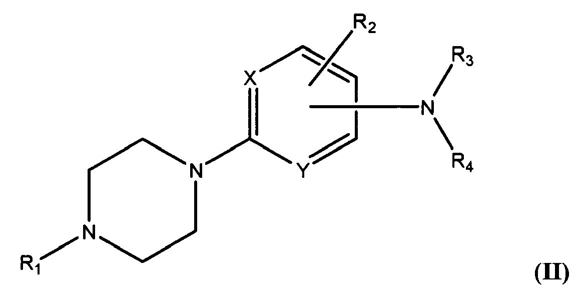 and pharmaceutically acceptable salts and/or esters thereof, wherein
- R₁ is hydrogen or substituted or unsubstituted alkyl;
- R₂ is hydrogen; halo; nitro; cyano, lower alkoxy; carboxylate salt acid or alkyl ester thereof; a sulfone; haloalkyl or haloalkoxy; alkylamide; acetaldehyde; carboxamide; carbonyl; alkoxyaminocarbonyl; or substituted arylalkylamino, wherein lower alkoxy is C₁₋₁₀ alkyl, C₂₋₅ alkenyl or C₂₋₅ alkynyl covalently linked to an oxygen atom;
- R₃ and R₄ independently are hydrogen, substituted or unsubstituted alkyl, aryl, alkylaryl, heteroaryl or alkylheteroaryl; or, taken together, R₃ and R₄ form one substituted or unsubstituted aryl, alkylaryl, heteroaryl or alkylheteroaryl group;
- and X and Y are each independently C or N; and
a second compound selected from the group consisting of metrifonate, neostigmine, physostigmine, pyridostigmine, galantamine/galanthamine, donepezil, tacrine, ambenonium, demarcarium, edrophonium, rivastigmine, phenserine, mentane, and eptastigmine; or pharmaceutically acceptable salts and/or esters thereof, wherein the second compound is present in an amount which, if administered to a patient alone, does not enhance memory.

3. A pharmaceutical composition comprising
a first compound having the formula 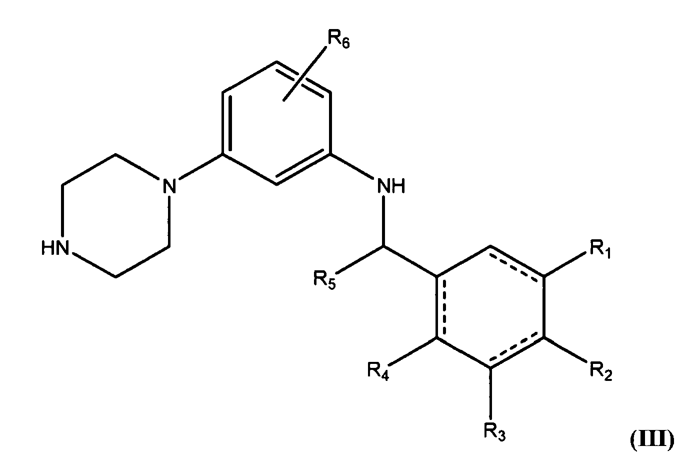 and pharmaceutically acceptable salts and/or esters thereof, wherein
- R₁, R₂, R₃ and R₄ independently are hydrogen, halo or lower alkoxy, or two adjoining R₁, R₂, R₃ or R₄ lower alkoxy groups may, taken with the benzyl ring to which they are attached, combine to form a ring;
- R₅ is hydrogen or lower alkoxy; and
- lower alkoxy is C₁₋₁₀alkyl, C₂₋₅ alkenyl or C₂₋₅ alkynyl covalently linked to an oxygen atom;
- R₆ is a sulfone, NO₂, or COCF₃; and
a second compound selected from the group consisting of metrifonate, neostigmine, physostigmine, pyridostigmine, galantamine/galanthamine, donepezil, tacrine, ambenonium, demarcarium, edrophonium, rivastigmine, phenserine, mentane, and eptastigmine; or pharmaceutically acceptable salts and/or esters thereof, wherein the second compound is present in an amount which, if administered to a patient alone, does not enhance memory.

4. A pharmaceutical composition comprising
■ a first compound having the formula 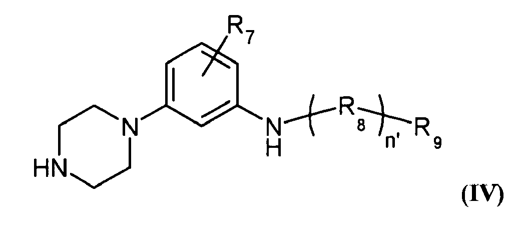 and pharmaceutically acceptable salts and/or esters thereof, wherein
- R₇ may be nitro, lower alkoxy; COCF₃ or C₁₋₁₀alkyl sulfonyl, wherein lower alkoxy is C₁₋₁₀ alkyl, C₂₋₅ alkenyl or C₂₋₅ alkynyl covalently linked to an oxygen atom;
- R₈ may be straight or branched C₁, C₂ or C₃ alkylene;
- n' is 0, 1 or 2;
- R₉ is substituted or unsubstituted substituted or unsubstituted aryl, naphthyl, chroman or tetrahydronaphthyl ring, wherein, when substituted, R₉ may have 1, 2, 3 or 4 substituents selected from C₁, C₂ or C₃ alkoxy; halo; or C₁, C₂ or C₃ alkyl, or when two substituents are present on adjacent carbon atoms, the substituents taken with the ring to which they are attached, may form a five to seven membered ring; and
a second compound selected from the group consisting of metrifonate, neostigmine, physostigmine, pyridostigmine, galantamine/galanthamine, donepezil, tacrine, ambenonium, demarcarium, edrophonium, rivastigmine, phenserine, mentane, and eptastigmine; or pharmaceutically acceptable salts and/or esters thereof, wherein the second compound is present in an amount which, if administered to a patient alone, does not enhance memory.

5. The pharmaceutical composition of any one of claims 1, 2 or 4 wherein the first compound is [1-(5-chloro-2,3-dimethoxy-phenyl)-ethyl]-(2-methanesulfonyl-5-piperazin-1-yl-phenyl)-amine, or a pharmaceutically acceptable salt and/or ester thereof.

6. The pharmaceutical composition of any one of claims 1-4, wherein the first compound is (2-(methylsulfonyl)-N-(1-phenylethyl)-5-(piperazin-1-yl)benzenamine; (1-(2-(1-(3,5-dimethoxyphenyl)ethylamino)-4-(piperazin-1-yl)phenyl)-2,2,2-trifluoroethanone; *N*-(1-(6-chloro-2,3-dihydrobenzo[b][1,4]dioxin-8-yl)ethyl)-2-(methylsulfonyl)-5-(piperazin-1-yl)benzenamine; *N* (3-chlorobenzyl)-2-nitro-5-(piperazin-1-yl)benzenamine; *N*-(3-chlorobenzyl)-4-nitro-3-(piperazin-1-yl)benzenamine; 1-(2-(1-(3,5-dimethoxyphenyl)ethylamino)-4-(piperazin-1-yl)phenyl)-2,2,2-trifluoroethanone; *N*-(1-(3-chlorophenyl)ethyl)-2-nitro-5-(piperazin-1-yl)benzenamine; *N*-(1-(3,5-dimethoxyphenyl)ethyl)-2-nitro-5-(piperazin-1-yl)benzenamine; 1-(2-(1-phenylethylamino)-4-(piperazin-1-yl)phenyl)-2,2,2-trifluoroethanone; 1-(2-(1-(3,5-dimethoxyphenyl)ethylamino)-4-(piperazin-1-yl)phenyl)-2,2,2-trifluoroethanone; 1-(2-(1-(3-methoxyphenyl)ethylamino)-4-(piperazin-1-yl)phenyl)-2,2,2-trifluoroethanone; 1-(2-(1-(3,5-dichlorophenyl)ethylamino)-4-(piperazin-1-yl)phenyl)-2,2,2-trifluoroethanone; *N*-(1-(5-chloro-2-methoxyphenyl)ethyl)-2-(methylsulfonyl)-5-(piperazin-1-yl)benzenamine; *N*-(2-methyl-1-phenylpropyl)-2-nitro-5-(piperazin-1-yl)benzenamine; 1-(2-(3,5-dimethoxybenzylamino)-4-(piperazin-1-yl)phenyl)-2,2,2-trifluoroethanone; *N*-(1-(3-bromophenyl)ethyl)-2-nitro-5-(piperazin-1-yl)benzenamine; 4-methoxy-*N*-(1-phenyl)ethyl)-3-(piperazin-1-yl)benzenamine; *N*-(3-bromobenzyl)-2-nitro-5-(piperazin-1-yl)benzenamine; *N*-(1-(3,5-dimethylyphenyl)ethyl)-2-nitro-5-(piperazin-1-yl)benzenamine; 1-(2-(3-bromobenzylamino)-4-(piperazin-1-yl)phenyl)-2,2,2-trifluoroethanone; (*S*)-1-(2-(1-phenylethylamino)-4-(piperazin-1-yl)phenyl)-2,2,2-trifluoroethanone; (*R*)-1-(2-(1-phenylethylamino)-4-(piperazin-1-yl)phenyl)-2,2,2-trifluoroethanone; *N*-(2-(methylsulfonyl)-5-(piperazin-1-yl)phenyl)naphthalen-1-amine hydrochloride; *N*-(5-fluoro-2-(methylsulfonyl)phenyl)-1,2,3,4-tetrahydronaphthalen-1-amine; or a pharmaceutically acceptable salt and/or ester thereof.

7. The pharmaceutical composition of any one of claims 1-6, wherein the first compound is present in an amount which if administered alone does not enhance memory.

8. The pharmaceutical composition of any one of claims 1-7, wherein said composition comprises a) about 10 mg to about 100 mg of the first compound; b) between about 100 mg and about 1000 mg of the first compound; or c) between about 1000 mg and 3000 mg of the first compound.

9. The pharmaceutical composition of any one of claims 1-8, wherein the second compound is donepezil, galantamine or rivastigmine.

10. The pharmaceutical composition of any one of claims 1-9, wherein the composition comprises between about 0.01 mg and 4.5 mg of the second compound.

11. The pharmaceutical composition of any one of claims 1 to 10 wherein the second compound is present in an amount sufficient to provide to a patient a dosage of less than 0.5mg/kg.

12. The pharmaceutical composition of any one of claims 1-11, wherein the first compound and the second compound are for administration in separate dosage forms or in a single dosage form.

13. The pharmaceutical composition of any one of claims 1-11, wherein the first compound and the second compound are for administration simultaneously or sequentially.

14. The pharmaceutical composition of any one of claims 1-13 for use in treating Alzheimer's disease or a cognition disorder.

15. The pharmaceutical composition of any one of claims 1-13 for use in enhancing memory.

16. A first compound having the formula (I), (II), (III) or (IV), or a pharmaceutically acceptable salt and/or ester thereof, as defined in any one of claims 1 to 4 in combination with a second compound selected from the group consisting of metrifonate, neostigmine, physostigmine, pyridostigmine, galantamine/galanthamine, donepezil, tacrine, ambenonium, demarcarium, edrophonium, rivastigmine, phenserine, mentane, and eptastigmine; or pharmaceutically acceptable salts and/or esters thereof, for use in treating Alzheimer's disease or a cognition disorder or enhancing memory, wherein the second compound is provided in an amount which, if administered to a patient alone, does not enhance memory.

17. The first and second compounds for use according to claim 16, wherein the first compound and the second compound are for administration in separate dosage forms or in a single dosage form.

18. The first and second compounds for use according to claim 16, wherein the first compound and the second compound are for administration simultaneously or sequentially.

19. The first and second compounds for use according to any one of claims 16 to 18, wherein the second compound is provided in an amount sufficient to provide to a patient a dosage of less than 0.5mg/kg.

20. The first and second compounds for use according to any one of claims 16 to 19, wherein the first compound is a compound as defined in claim 5 or 6, or a pharmaceutically acceptable salt and/or ester thereof.

## Patentansprüche

1. Pharmazeutische Zusammensetzung, welche Folgendes umfasst:
• eine erste Verbindung mit der Formel 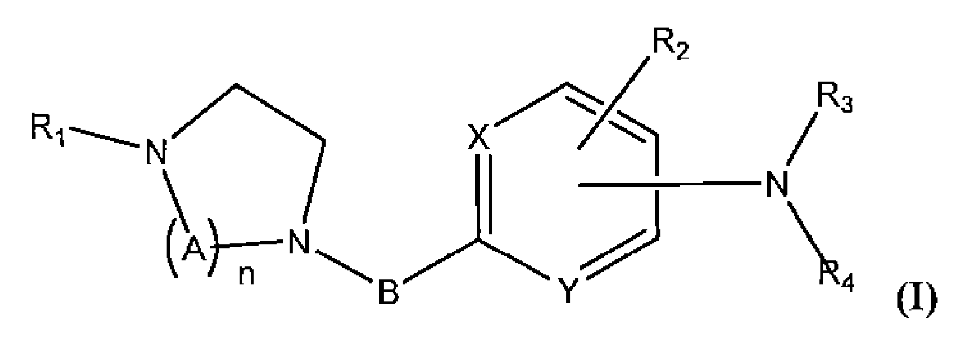 und pharmazeutisch akzeptable Salze und/oder Ester davon, wobei
- n 0, 1, 2, 3 oder 4 ist,
- A, wenn vorhanden, eine C1-10-Alkylgruppe ist,
- R₁ Wasserstoff oder ein substituiertes oder nicht substituiertes Alkyl oder Aryl ist,
- R₂ Wasserstoff oder Halogen, Nitro, Cyano, ein niederes Alkoxy, eine Carboxylatsalz-Säure oder ein Alkylester davon, ein Sulfon, Halogenalkyl oder Halogenalkoxy, Acetaldehyd, Carboxamid, Carbonyl, Alkoxyaminocarbonyl oder substitutiertes Arylalkylamino ist, wobei niederes Alkoxy C₁₋₁₀-Alkyl, C₂₋₅-Alkenyl oder C₂₋₅-Alkynyl ist, kovalent mit einem Sauerstoffatom verknüpft
- R₃ und R₄ unabhängig Wasserstoff, ein substituiertes oder nicht substituiertes Alkyl, Aryl, Alkylaryl, Heteroaryl oder Alkylheteroaryl sind oder R₃ und R₄, zusammen genommen, eine substituierte oder nicht substituierte Aryl-, Alkylaryl-, Heteroaryl- oder Alkylheteroarylgruppe bilden,
- B vorhanden oder nicht vorhanden sein kann und, wenn vorhanden, C₁₋₁₀-Alkyl ist, und
- X und Y unabhängig C oder N sind, und
eine zweite Verbindung, die aus der Gruppe ausgewählt ist, die aus Metrifonat, Neostigmin, Physostigmin, Pyridostigmin, Galantamin/Galanthamin, Donepezil, Tacrin, Ambenonium, Demarcarium, Edrophonium, Rivastigmin, Phenserin, Menthan und Eptastigmin besteht, oder pharmazeutisch akzeptable Salze und/oder Ester davon, wobei die zweite Verbindung in einer Menge vorhanden ist, die, wenn sie einem Patienten allein verabreicht wird, das Gedächtnis nicht verbessert.

2. Pharmazeutische Zusammensetzung, welche Folgendes umfasst:
eine erste Verbindung mit der Formel 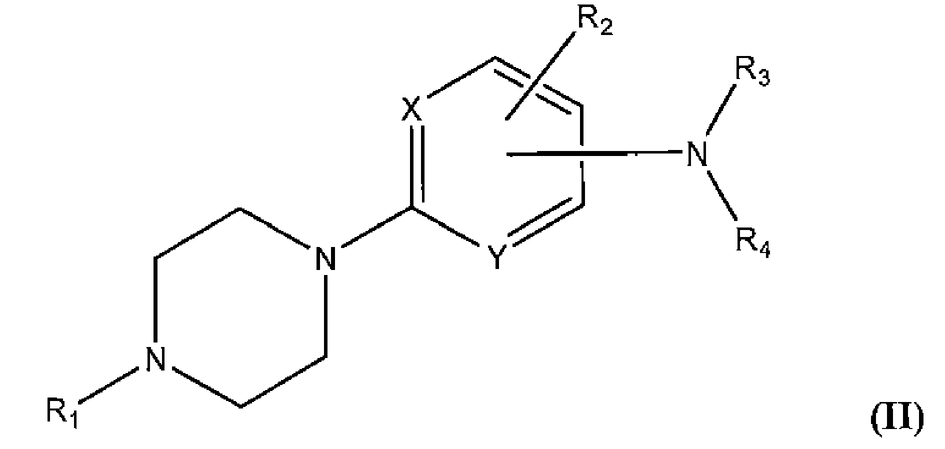 und pharmazeutisch akzeptable Salze und/oder Ester davon, wobei
- R₁ Wasserstoff oder ein substituiertes oder nicht substituiertes Alkyl ist,
- R₂ Wasserstoff, Halogen, Nitro, Cyano, ein niederes Alkoxy, eine Carboxylatsalz-Säure oder ein Alkylester davon, ein Sulfon, Halogenalkyl oder Halogenalkoxy, Alkylamid, Acetaldehyd, Carboxamid, Carbonyl, Alkoxyaminocarbonyl oder substitutiertes Arylalkylamino ist, wobei niederes Alkoxy C₁₋₁₀-Alkyl, C₂₋₅-Alkenyl oder C₂₋₅-Alkynyl ist, kovalent mit einem Sauerstoffatom verknüpft,
- R₃ und R₄ unabhängig Wasserstoff, ein substituiertes oder nicht substituiertes Alkyl, Aryl, Alkylaryl, Heteroaryl oder Alkylheteroaryl sind oder R₃ und R₄, zusammen genommen, eine substituierte oder nicht substituierte Aryl-, Alkylaryl-, Heteroaryl- oder Alkylheteroarylgruppe bilden,
- und X und Y jeweils unabhängig C oder N sind, und
eine zweite Verbindung, die aus der Gruppe ausgewählt ist, die aus Metrifonat, Neostigmin, Physostigmin, Pyridostigmin, Galantamin/Galanthamin, Donepezil, Tacrin, Ambenonium, Demarcarium, Edrophonium, Rivastigmin, Phenserin, Menthan und Eptastigmin besteht, oder pharmazeutisch akzeptable Salze und/oder Ester davon, wobei die zweite Verbindung in einer Menge vorhanden ist, die, wenn sie einem Patienten allein verabreicht wird, das Gedächtnis nicht verbessert.

3. Pharmazeutische Zusammensetzung, welche Folgendes umfasst:
eine erste Verbindung mit der Formel 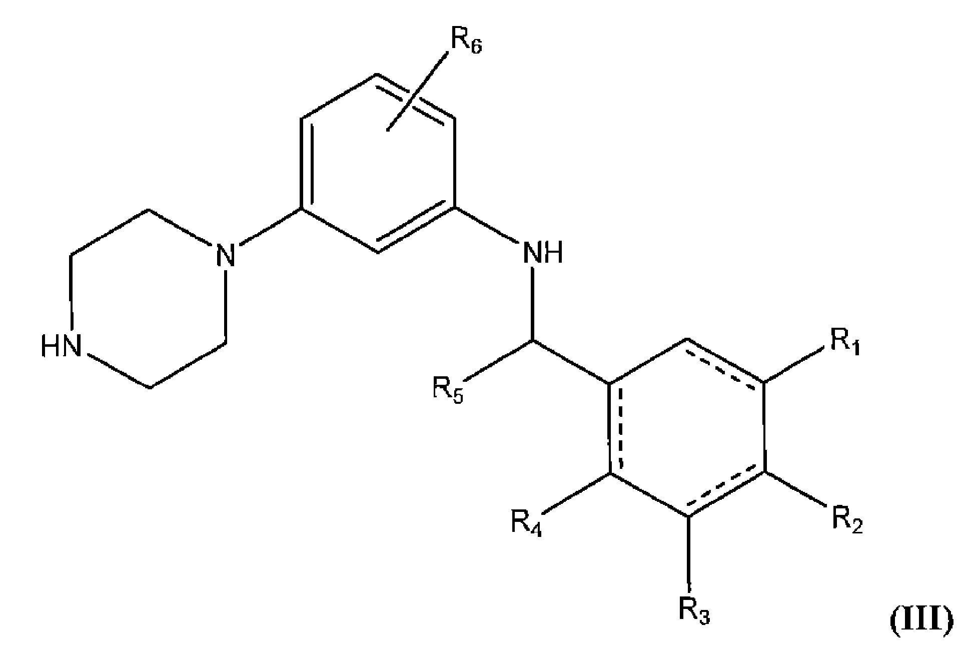 und pharmazeutisch akzeptable Salze und/oder Ester davon, wobei
- R₁, R₂, R₃ und R₄ unabhängig Wasserstoff, Halogen oder ein niederes Alkoxy sind, oder sich zwei benachbarte niedere R₁-, R₂-, R₃- oder R₄-Alkoxygruppen, zusammen mit dem Benzylring, an dem sie befestigt sind, kombinieren, um einen Ring zu bilden;
- R₅ Wasserstoff oder ein niederes Alkoxy ist und
- niederes Alkoxy C₁₋₁₀-Alkyl, C₂₋₅-Alkenyl oder C₂₋₅-Alkynyl ist, kovalent mit einem Sauerstoffatom verknüpft,
- R₆ ein Sulfon, NO₂ oder COCF₃ ist und
eine zweite Verbindung, die aus der Gruppe ausgewählt ist, die aus Metrifonat, Neostigmin, Physostigmin, Pyridostigmin, Galantamin/Galanthamin, Donepezil, Tacrin, Ambenonium, Demarcarium, Edrophonium, Rivastigmin, Phenserin, Menthan und Eptastigmin besteht, oder pharmazeutisch akzeptable Salze und/oder Ester davon, wobei die zweite Verbindung in einer Menge vorhanden ist, die, wenn sie einem Patienten allein verabreicht wird, das Gedächtnis nicht verbessert.

4. Pharmazeutische Zusammensetzung, welche Folgendes umfasst:
• eine erste Verbindung mit der Formel 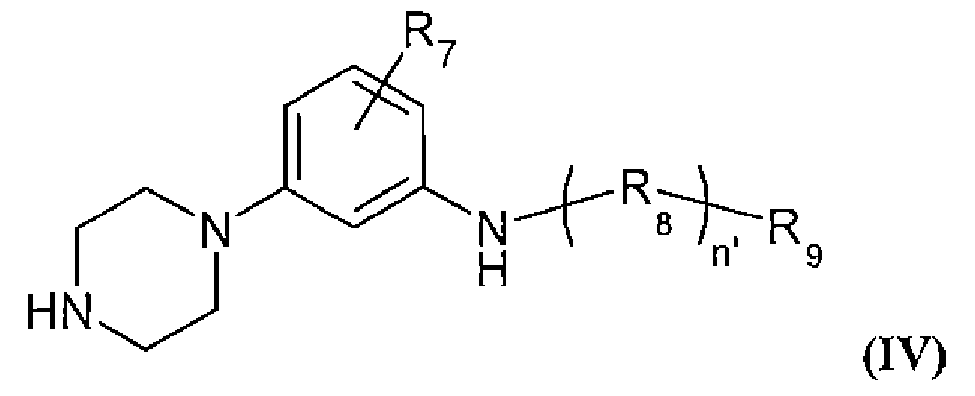 und pharmazeutisch akzeptable Salze und/oder Ester davon, wobei
- R₇ Nitro, niederes Alkoxy, COCF₃ oder C₁₋₁₀-Alkylsulfonyl ist, wobei niederes Alkoxy C₁₋₁₀-Alkyl, C₂₋₅-Alkenyl oder C₂₋₅-Alkynyl ist, kovalent mit einem Sauerstoffatom verknüpft,
- R₈ gerades oder verzweigtes C₁-, C₂- oder C₃-Alkylen sein kann,
- n' 0, 1 oder 2 ist;
- R₉ ein substituierter oder nicht substituierter Aryl-, Naphthyl-, Chroman- oder Tetrahydronaphthylring ist, wobei, wenn substituiert, R₉ 1, 2, 3 oder 4 Substituenten aufweisen kann, die aus C₁-, C₂- oder C₃-Alkoxy, Halogen oder C₁-, C₂- oder C₃-Alkyl ausgewählt sind, oder, wenn zwei Substituenten an benachbarten Kohlenstoffatomen vorhanden sind, die Substituenten zusammen mit dem Ring, an dem sie befestigt sind, einen fünf- bis siebengliedrigen Ring bilden können und
eine zweite Verbindung, die aus der Gruppe ausgewählt ist, die aus Metrifonat, Neostigmin, Physostigmin, Pyridostigmin, Galantamin/Galanthamin, Donepezil, Tacrin, Ambenonium, Demarcarium, Edrophonium, Rivastigmin, Phenserin, Menthan und Eptastigmin besteht, oder pharmazeutisch akzeptable Salze und/oder Ester davon, wobei die zweite Verbindung in einer Menge vorhanden ist, die, wenn sie einem Patienten allein verabreicht wird, das Gedächtnis nicht verbessert.

5. Pharmazeutische Verbindung nach einem der Ansprüche 1, 2 oder 4, wobei die erste Verbindung [1-(5-Chlor-2,3-dimethoxy-phenyl)-ethyl]-(2-methanesulfonyl-S-piperazin-1-yl-phenyl)-amin oder ein pharmazeutisch akzeptables Salz und/oder Ester davon ist.

6. Pharmazeutische Verbindung nach einem der Ansprüche 1-4, wobei die erste Verbindung (2-(Methylsulfonyl)-N-(1-phenylethyl)-5- (piperazin-1-yl)benzolamin, (1-(2-(1-(3, 5-Dimethoxyphenyl)ethylamino)-4-(piperazin-1-yl)phenyl)-2,2,2-trifluorethanon, N-(1-(6-Chlor-2,3-dihydrobenzo[b][1,4]dioxin-8-yl)ethyl)-2-(methylsulfonyl)-5-(piperazin-1-yl)benzolamin, N-(3-Chlorbenzyl)-2-nitro-5-(piperazin-1-yl)benzolamin, N-(3-Chlorbenzyl)-4-nitro-3-(piperazin-1-yl)benzolamin, 1-(2-(1-(3,5-Dimethoxyphenyl)ethylamino)-4-(piperazin-1-yl)phenyl)-2,2,2-trifluorethanon, N-(1-(3-Chlorphenyl)ethyl)-2-nitro-5-(piperazin-1-yl)benzolamin, N-(1-(3,5-Dimethoxyphenyl)ethyl)-2-nitro-5-(piperazin-1-yl)benzolamin, 1-(2-(1-Phenylethylamino)-4-(piperazin-1-yl)phenyl)-2,2,2-trifluorethanon, 1-(2-(1-(3,5-Dimethoxyphenyl)ethylamino)-4-(piperazin-1-yl)phenyl)-2,2,2-trifluorethanon, 1-(2-(1-(3-Methoxyphenyl)-ethylamino)-4-(piperazin-1-yl)phenyl)-2,2,2-trifluorethanon, 1-(2-(1-(3,5-Dichlorphenyl)ethylamino)-4-(piperazin-1-yl)phenyl)-2,2,2-trifluorethanon, N-(1-(5-Chlor-2-methoxyphenyl)ethyl)-2-(methylsulfonyl)-5-(piperazin-1-yl)benzolamin, N-(2-Methyl-1-phenylpropyl)-2-nitro-5-(piperazin-1-yl)benzolamin, 1-(2-(3,5-Dimethoxybenzylamino)-4-(piperazin-1-yl)phenyl)-2,2,2-trifluorethanon, N-(1-(3-Bromphenyl)-ethyl)-2-nitro-5-(piperazin-1-yl)benzolamin, 4-Methoxy-N-(1-phenyl)ethyl)-3-(piperazin-1-yl)benzolamin, N-(3-Brombenzyl)-2-nitro-5-(piperazin-1-yl)benzolamin, N-(1-(3,5-Dimethylyphenyl)ethyl)-2-nitro-5-(piperazin-1-yl)benzolamin, 1-(2-(3-Brombenzylamino)-4-(piperazin-1-yl)-phenyl)-2,2,2-trifluorethanon, (S)-1-(2-(1-Phenylethylamino)-4-(piperazin-1-yl)phenyl)-2,2,2-trifluorethanon, (R)-1-(2-(1-Phenylethylamino)-4-(piperazin-1-yl)phenyl)-2,2,2-trifluorethanon, N-(2-(Methylsulfonyl)-5-(piperazin-1-yl)phenyl)naphthalen-1-aminhydrochlorid, N-(5-Fluor-2-(methylsulfonyl)-phenyl)-1,2,3,4-tetrahydronaphthalen-1-amin oder ein pharmazeutisch akzeptables Salz und/oder Ester davon ist.

7. Pharmazeutische Zusammensetzung nach einem der Ansprüche 1-6, wobei die erste Verbindung in einer Menge vorhanden ist, die, wenn sie allein verabreicht wird, das Gedächtnis nicht verbessert.

8. Pharmazeutische Zusammensetzung nach einem der Ansprüche 1-7, wobei die Zusammensetzung a) etwa 10 mg bis etwa 100 mg der ersten Verbindung, b) zwischen etwa 100 mg und etwa 1000 mg der ersten Verbindung oder c) zwischen etwa 1000 mg und 3000 mg der ersten Verbindung enthält.

9. Pharmazeutische Zusammensetzung nach einem der Ansprüche 1-8, wobei die zweite Verbindung Donepezil, Galantamin oder Rivastigmin ist.

10. Pharmazeutische Zusammensetzung nach einem der Ansprüche 1-9, wobei die Zusammensetzung zwischen etwa 0,01 mg und 4,5 mg der zweiten Verbindung enthält.

11. Pharmazeutische Zusammensetzung nach einem der Ansprüche 1 bis 10, wobei die zweite Verbindung in einer Menge vorhanden ist, die ausreichend ist, um einen Patienten mit einer Dosierung von weniger als 0,5 mg/kg zu versehen.

12. Pharmazeutische Zusammensetzung nach einem der Ansprüche 1-11, wobei die erste Verbindung und die zweite Verbindung für die Verabreichung in separaten Dosierungsformen oder in einer einzelnen Dosierungsform bestimmt sind.

13. Pharmazeutische Zusammensetzung nach einem der Ansprüche 1-11, wobei die erste Verbindung und die zweite Verbindung für die simultane oder aufeinander folgende Verabreichung bestimmt sind.

14. Pharmazeutische Zusammensetzung nach einem der Ansprüche 1-13 zur Verwendung beim Behandeln von Alzheimer-Krankheit oder einer Kognitionsstörung.

15. Pharmazeutische Zusammensetzung nach einem der Ansprüche 1-13 zur Verwendung beim Verbessern des Gedächtnisses.

16. Erste Verbindung, welche die Formel (I), (II), (III) oder (IV) aufweist, oder ein pharmazeutisch akzeptables Salz und/oder ein pharmazeutisch akzeptabler Ester davon, wie in einem der Ansprüche 1 bis 4 definiert, für die Verwendung in Kombination mit einer zweiten Verbindung, die aus der Gruppe ausgewählt ist, die aus Metrifonat, Neostigmin, Physostigmin, Pyridostigmin, Galantamin/Galanthamin, Donepezil, Tacrin, Ambenonium, Demarcarium, Edrophonium, Rivastigmin, Phenserin, Menthan und Eptastigmin besteht, oder pharmazeutisch akzeptablen Salzen und/oder Estern davon, zum Behandeln von Alzheimer-Krankheit oder einer Kognitionsstörung oder zum Verbessern des Gedächtnisses, wobei die zweite Verbindung in einer Menge versehen wird, die, wenn sie einem Patienten allein verabreicht wird, das Gedächtnis nicht verbessert.

17. Erste Verbindung für die Verwendung nach Anspruch 16, wobei die erste Verbindung und die zweite Verbindung für die Verabreichung in separaten Dosierungsformen oder in einer einzelnen Dosierungsform bestimmt sind.

18. Erste Verbindung für die Verwendung nach Anspruch 16, wobei die erste Verbindung und die zweite Verbindung für die simultane oder aufeinander folgende Verabreichung bestimmt sind.

19. Erste Verbindung für die Verwendung nach einem der Ansprüche 16 bis 18, wobei die zweite Verbindung in einer Menge versehen wird, die ausreichend ist, um einen Patienten mit einer Dosierung von weniger als 0,5 mg/kg zu versehen.

20. Erste Verbindung für die Verwendung nach einem der Ansprüche 16 bis 19, wobei es sich bei der ersten Verbindung um eine Verbindung wie in Anspruch 5 oder 6 definiert oder um ein pharmazeutisch akzeptables Salz und/oder einen pharmazeutisch akzeptablen Ester davon handelt.

## Revendications

1. Composition pharmaceutique comprenant
• un premier composé de formule 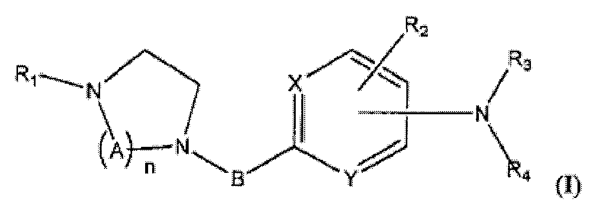 et ses sels et/ou esters pharmaceutiquement acceptables, dans laquelle
- n est 0, 1, 2, 3 ou 4 ;
- A, s'il est présent, est un groupe alkyle en C₁ à C₁₀ ;
- R₁ est un atome d'hydrogène ou un groupe alkyle ou aryle substitué ou non substitué :
- R₂ est un atome d'hydrogène ; un groupe halogéno ; nitro ; cyano, alcoxy inférieur ; un acide de sel carboxylate ou un ester d'alkyle de celui-ci ; une sulfone ; un groupe halogénoalkyle ou halogéno-alcoxy ; acétaldéhyde ; carboxamide ; carbonyle ; alcoxyaminocarbonyle ; ou arylalkylamino substitué ; le groupe alcoxy inférieur étant un groupe alkyle en C₁ à C₁₀, alcényle en C₂ à C₅ ou alcynyle en C₂ à C₅ lié de façon covalente à un atome d'oxygène ;
- R₃ et R₄ sont indépendamment un atome d'hydrogène, un groupe alkyle substitué ou non substitué, aryle, alkylaryle, hétéroaryle ou alkylhétéroaryle ou pris conjointement, R₃ et R₄ forment un groupe aryle substitué ou non substitué, alkylaryle, hétéroaryle ou alkylhétéroaryle ;
- B peut être présent ou absent et s'il est présent, il est un groupe alkyle en C₁ à C₁₀ ; et
- X et Y sont indépendamment C ou N ; et
• un deuxième composé choisi dans le groupe comprenant le métrifonate, la néostigmine, la physostigmine, la pyridostigmine, la galantamine/galanthamine, le donépézil, le tacrine, l'ambénonium, le démarcarium, l'édrophonium, la rivastigmine, la phensérine, le menthane et l'eptastigmine ; ou des sels et/ou esters pharmaceutiquement acceptables de ceux-ci, le deuxième composé étant présent en une quantité qui, s'il est administré à un patient seul, ne renforce pas la mémoire.

2. Composition pharmaceutique comprenant
• un premier composé de formule 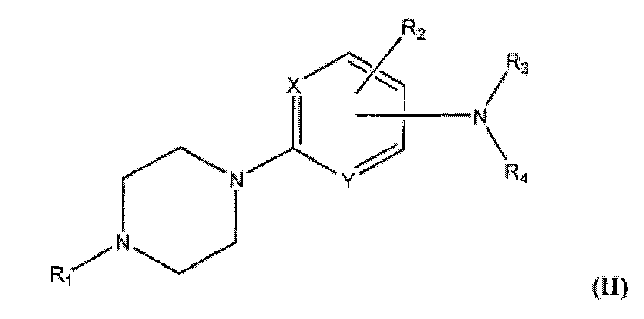 et ses sels et/ou esters pharmaceutiquement acceptables, dans laquelle
- R₁ est un atome d'hydrogène ou un groupe alkyle substitué ou non substitué :
- R₂ est un atome d'hydrogène ; un groupe halogéno ; nitro ; cyano, alcoxy inférieur ; un acide de sel carboxylate ou un ester d'alkyle de celui-ci ; une sulfone ; un groupe halogénoalkyle ou halogéno-alcoxy ; alkylamide, acétaldéhyde ; carboxamide ; carbonyle ; alcoxyaminocarbonyle ; ou arylalkylamino substitué ; le groupe alcoxy inférieur étant un groupe alkyle en C₁ à C₁₀, alcényle en C₂ à C₅ ou alcynyle en C₂ à C₅ lié de façon covalente à un atome d'oxygène ;
- et R₃ et R₄ sont indépendamment un atome d'hydrogène, un groupe alkyle substitué ou non substitué, aryle, alkylaryle, hétéroaryle ou alkylhétéroaryle ou pris conjointement, R₃ et R₄ forment un groupe aryle substitué ou non substitué, alkylaryle, hétéroaryle ou alkylhétéroaryle,
- et X et Y sont chacun indépendamment, C ou N ; et
• un deuxième composé choisi dans le groupe comprenant le métrifonate, la néostigmine, la physostigmine, la pyridostigmine, la galantamine/galanthamine, le donépézil, le tacrine, l'ambénonium, le démarcarium, l'édrophonium, la rivastigmine, la phensérine, le menthane et l'eptastigmine ; ou des sels et/ou esters pharmaceutiquement acceptables de ceux-ci, le deuxième composé étant présent en une quantité qui, s'il est administré à un patient seul, ne renforce pas la mémoire.

3. Composition pharmaceutique comprenant
• un premier composé de formule 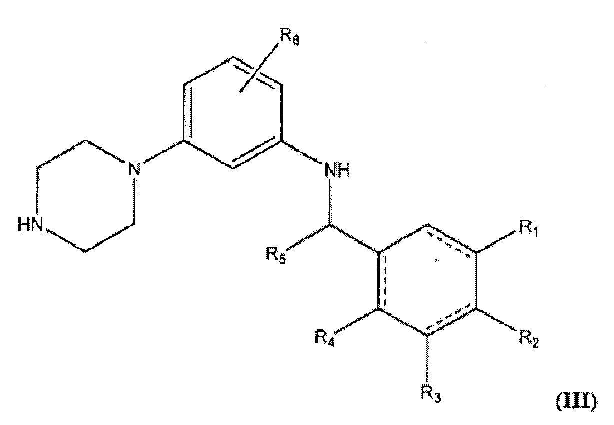 et ses sels et/ou esters pharmaceutiquement acceptables, dans laquelle
- R₁, R₂, R₃ et R₄ sont indépendamment un atome d'hydrogène, un groupe halogéno ou alcoxy inférieur ou deux groupes alcoxy inférieurs attenant à R₁, R₂, R₃ ou R₄, pris conjointement avec le cycle benzyle auquel ils sont liés, peuvent se combiner pour former un cycle ;
- R₅ est un atome d'hydrogène ou un groupe alcoxy inférieur ; et
- le groupe alcoxy inférieur est un groupe alkyle en C₁ à C₁₀, alcényle en C₂ à C₅ ou alcynyle en C₂ à C₅ liés de façon covalente à un atome d'oxygène ;
- R₆ est une sulfone, NO₂ ou COCF₃ ; et
• un deuxième composé choisi dans le groupe comprenant le métrifonate, la néostigmine, la physostigmine, la pyridostigmine, la galantamine/galanthamine, le donépézil, le tacrine, l'ambénonium, le démarcarium, l'édrophonium, la rivastigmine, la phensérine, le menthane et l'eptastigmine ; ou des sels et/ou esters pharmaceutiquement acceptables de ceux-ci, le deuxième composé étant présent en une quantité qui, s'il est administré à un patient seul, ne renforce pas la mémoire.

4. Composition pharmaceutique comprenant
• un premier composé de formule 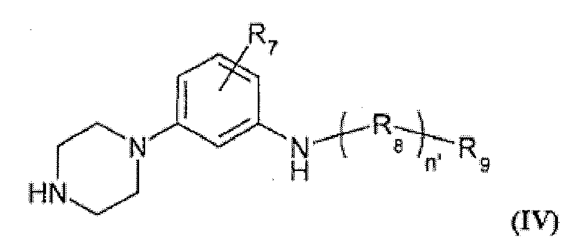 et des sels et/ou esters pharmaceutiquement acceptables de ceux-ci, dans laquelle
- R₇ peut être un groupe nitro, alcoxy inférieur ; COCF₃ ou alkylsulfonyle en C₁ à C₁₀, le groupe alcoxy inférieur étant un groupe alkyle en C₁ à C₁₀, alcényle en C₂ à C₅ ou alcynyle en C₂ à C₅ lié de façon covalente à un atome d'oxygène ;
- R₈ peut être un groupe alkylène en C₁, C₂ ou C₃, linéaire ou ramifié ;
- n' est 0, 1 ou 2 ;
- R₉ est un cycle aryle substitué ou non substitué, naphtyle, chromane ou tétrahydronaphtyle, dans lequel, s'il est substitué, R₉ peut avoir 1, 2, 3 ou 4 substituants choisis parmi un groupe alcoxy en C₁, C₂ ou C₃ ; un groupe halogéno ; ou alkyle en C₁, C₂ ou C₃ ou si deux substituants sont présents sur des atomes de carbone adjacents, les substituants pris avec le cycle auquel ils sont liés peuvent former un cycle de cinq à sept chaînons ; et
• un deuxième composé choisi dans le groupe comprenant le métrifonate, la néostigmine, la physostigmine, la pyridostigmine, la galantamine/galanthamine, le donépézil, le tacrine, l'ambénonium, le démarcarium, l'édrophonium, la rivastigmine, la phensérine, le menthane et l'eptastigmine ; ou des sels et/ou esters pharmaceutiquement acceptables de ceux-ci, le deuxième composé étant présent en une quantité qui, s'il est administré à un patient seul, ne renforce pas la mémoire.

5. Composition pharmaceutique selon l'une quelconque des revendications 1,2 ou 4, dans laquelle le premier composé est la [1-(5-chloro-2,3-diméthoxy-phényl)-éthyl]-(2-méthanesulfonyl-5-pipérazin-1-yl-phényl)-amine ou un sel et/ou ester pharmaceutiquement acceptable de celle-ci.

6. Composition pharmaceutique selon l'une quelconque des revendications 1 à 4, dans laquelle le premier composé est la (2-(méthylsulfonyl)-N-(1-phényléthyl)-5-(pipérazin-1-yl)benzène-amine ; la (1-(2-(1-(3,5-diméthoxyphényl)éthylamino)-4-(pipérazin-1-yl)phényl-2,2,2-trifluoro-éthanone ; la N-(1-(6-chloro-2,3-dihydrobenzo[b][1,4]dioxin-8-yl)éthyl)-2-(méthylsulfonyl)-5-(pipérazin-1-yl)benzène-amine ; la N-(3-chlorobenzyl)-2-nitro-5-(pipérazin-1-yl)benzène-amine ; la N-(3-chlorobenzyl)-4-nitro-3-(pipérazin-1-yl)benzène-amine ; la 1-(2-(1-(3,5-diméthoxyphényl) éthylamino)-4-(pipérazin-1-yl)phényl-2,2,2-trifluoroéthanone ; la N-(1-(3-chlorophényl)éthyl)-2-nitro-5-(pipérazin-1-yl)benzène-amine ; la N-(1-(3,5-diméthoxyphényl)éthyl)-2-nitro-5-(pipérazin-1-yl) benzène-amine ; la 1-(2-(1-phényléthylamino)-4-(pipérazin-1-yl)phényl-2,2,2-trifluoroéthanone ; la 1-(2-(1-(3,5-diméthoxyphényl) éthylamino)-4-(pipérazin-1-yl)phényl)-2,2,2-trifluoro-éthanone ; la 1-(2-(1-(3-méthoxyphényl)éthylamino)-4-pipérazin-1-yl)phényl-2,2,2-trifluoroéthanone ; la 1-(2-(1-(3,5-dichlorophényl)éthylamino)-4-(pipérazin-1-yl) phényl)-2,2,2-trifluoroéthanone ; la N-(1-(5-chloro-2-méthoxyphényl)éthyl)-2-(méthylsulfonyl)-5-pipérazin-1-yl)benzamine ; la N-(2-méthyl-1-phénylpropyl)-2-nitro-5-(pipérazin-1-yl)benzène-amine ; la 1-(2-(3,5-diméthoxy-benzylamino)-4-(pipérazin-1-yl)phényl)-2,2,2-trifluoroéthanone ; la N-(1-(3-bromophényl)éthyl)-2-nitro-5-(pipérazin-1-yl)benzène-amine ; la 4-méthoxy-N-(1-phényl)éthyl)-3-(pipérazin-1-yl)benzène-amine ; la N-(3-bromobenzyl)-2-nitro-5-(pipérazin-1-yl)benzène-amine ; la N-(1-(3,5-diméthylphényl)éthyl)-2-nitro-5-(pipérazin-1-yl)benzène-amine ; la 1-(2-(3-bromobenzylamino)-4-(pipérazin-1-yl)phényl)-2,2,2-trifluoro-éthanone ; la (S)-1-(2-(1-phényléthylamino)-4-(pipérazin-1-yl)phényl-2,2,2-trifluoro-éthanone ; la (R)-1-(2-(1-phényléthyl-amino)-4-(pipérazin-1-yl)phényl)-2,2,2-trifluoroéthanone ; le chlorhydrate de N-(2-(méthylsulfonyl)-5-(pipérazin-1-yl)phényl)naphtalén-1-amine ; la N-(5-fluoro-2-(méthylsulfonyl)phényl)-1,2,3,4-tétrahydronaphtalén-1-amine ; ou un sel et/ou ester pharmaceutiquement acceptable de ceux-ci.

7. Composition pharmaceutique selon l'une quelconque des revendications 1 à 6, dans laquelle le premier composé est présent en une quantité qui, s'il est administré seul, ne renforce pas la mémoire.

8. Composition pharmaceutique selon l'une quelconque des revendications 1 à 7, ladite composition comprenant a) environ 10 mg à environ 100 mg du premier composé ; b) entre environ 100 mg et environ 1000 mg du premier composé ; ou c) entre environ 1000 mg et 3000 mg du premier composé.

9. Composition pharmaceutique selon l'une quelconque des revendications 1 à 8, dans laquelle le deuxième composé est le donépézil, la galantamine ou la rivastigmine.

10. Composition pharmaceutique selon l'une quelconque des revendications 1 à 9, ladite composition comprenant entre environ 0,01 mg et 4,5 mg du deuxième composé.

11. Composition pharmaceutique selon l'une quelconque des revendications 1 à 10, dans laquelle le deuxième composé est présent en une quantité suffisante pour fournir au patient une dose inférieure à 0,5 mg/kg.

12. Composition pharmaceutique selon l'une quelconque des revendications 1 à 11, dans laquelle le premier composé et le deuxième composé sont destinés à une administration en formes galéniques séparées ou en une forme galénique unique.

13. Composition pharmaceutique selon l'une quelconque des revendications 1 à 11, dans laquelle le premier composé et le deuxième composé sont destinés à une administration simultanée ou séquentielle.

14. Composition pharmaceutique selon l'une quelconque des revendications 1 à 13, à utiliser dans le traitement de la maladie d'Alzheimer ou d'un trouble cognitif.

15. Composition pharmaceutique selon l'une quelconque des revendications 1 à 13, à utiliser pour améliorer la mémoire.

16. Premier composé de formule (I), (II), (III) ou (IV) ou sel et/ou ester de celui-ci pharmaceutiquement acceptable selon l'une quelconque des revendications 1 à 4 à utiliser en combinaison avec un deuxième composé choisi dans le groupe comprenant le métrifonate, la néostigmine, la physostigmine, la pyridostigmine, la galantamine/galanthamine, le donépézil, le tacrine, l'ambénonium, le démarcarium, l'édrophonium, la rivastigmine, la phensérine, le menthane et l'eptastigmine ; ou des sels et/ou esters pharmaceutiquement acceptables de ceux-ci, dans le traitement de la maladie d'Alzheimer ou d'un trouble cognitif ou pour renforcer la mémoire, le deuxième composé étant fourni en une quantité qui, si elle est administrée à un patient seule, ne renforce pas la mémoire.

17. Premier composé à utiliser selon la revendication 16, le premier composé et le deuxième composé étant destiné à une administration en formes galéniques séparées ou en une forme galénique unique.

18. Premier composé à utiliser selon la revendication 16, le premier composé et le deuxième composé étant destinés à l'administration simultanée ou séquentielle.

19. Premier composé à utiliser selon l'une quelconque des revendications 16 à 18, le deuxième composé étant fourni en une quantité suffisante pour fournir à un patient une dose inférieure à 0,5 mg/kg.

20. Premier composé à utiliser selon l'une quelconque des revendications 16 à 19, le premier composé étant un composé tel que défini dans la revendication 5 ou 6, ou un sel et/ou ester pharmaceutiquement acceptable de celui-ci.
